# EUROPEAN PATENT SPECIFICATION

(11) **EP 3 605 629 B1**
(45) Date of publication and mention of the grant of the patent: **18.05.2022**
(21) Application number: 18775969.1
(22) Date of filing: 23.03.2018
(51) Int. Cl.: H01L 51/30, C08L 65/00, C07D 333/50, H01L 21/336, C07D 495/22, H01L 29/786, C08G 61/12, H01L 51/05, C08K 5/34

(54) **ORGANIC SEMICONDUCTOR ELEMENT, ORGANIC SEMICONDUCTOR COMPOSITION, ORGANIC SEMICONDUCTOR FILM PRODUCTION METHOD, ORGANIC SEMICONDUCTOR FILM, AND COMPOUND AND POLYMER USED THEREFOR**
ORGANISCHES HALBLEITERELEMENT, ORGANISCHE HALBLEITERZUSAMMENSETZUNG, ORGANISCHES HALBLEITERFILMHERSTELLUNGSVERFAHREN, ORGANISCHER HALBLEITERFILM UND VERBINDUNG UND POLYMER DAFÜR
ÉLÉMENT SEMI-CONDUCTEUR ORGANIQUE, COMPOSITION SEMI-CONDUCTRICE ORGANIQUE, PROCÉDÉ DE PRODUCTION DE FILM SEMI-CONDUCTEUR ORGANIQUE, FILM SEMI-CONDUCTEUR ORGANIQUE, ET COMPOSÉ ET POLYMÈRE UTILISÉS À CET EFFET

(30) Priority: 31.03.2017 JP 2017071817
(43) Date of publication of application: 05.02.2020
(73) Proprietor: FUJIFILM Corporation, Tokyo 106-8620 (JP); The University Of Tokyo, Tokyo 113-8654 (JP)
(72) Inventor: SHIROKANE, Kenji, Ashigarakami-gun Kanagawa 258-8577 (JP); FUKUZAKI, Eiji, Ashigarakami-gun Kanagawa 258-8577 (JP); TANI, Yukio, Ashigarakami-gun Kanagawa 258-8577 (JP); TAMAKUNI, Fumiko, Ashigarakami-gun Kanagawa 258-8577 (JP); USAMI, Yoshihisa, Ashigarakami-gun Kanagawa 258-8577 (JP); WATANABE, Tetsuya, Ashigarakami-gun Kanagawa 258-8577 (JP); OKAMOTO, Toshihiro, Tokyo 113-8654 (JP); TAKEYA, Junichi, Tokyo 113-8654 (JP)
(74) Representative: Hoffmann Eitle
(86) International application number: PCT/JP2018/011873
(87) International publication number: WO 2018/181054

(56) References cited:
- WO-A1-2014/156878
- WO-A1-2017/022735
- JP-A- H11 149 984
- JP-A- 2015 192 118
- KR-A- 20140 091 487
- YAMANE ET AL: "Organic heterojunction ambipolar field effect transistors with asymmetric source and drain electrodes", THIN SOLID FILMS, ELSEVIER, AMSTERDAM, NL, vol. 516, no. 9, 22 February 2008 (2008-02-22), pages 2758-2761, XP022494828, ISSN: 0040-6090, DOI: 10.1016/J.TSF.2007.04.117

## Description

### BACKGROUND OF THE INVENTION

### 1. Field of the Invention

The present invention relates to an organic semiconductor element, an organic semiconductor composition, a method of manufacturing an organic semiconductor film, an organic semiconductor film, and a compound.

### 2. Description of the Related Art

A semiconductor element is used in a liquid crystal display, and a display such as an organic electroluminescent display, a radio frequency identifier (RFID: RF tags), a logic circuit such as a memory, and a solar cell. Among these, an organic semiconductor element having an organic semiconductor film is superior to an inorganic semiconductor element having an inorganic semiconductor film, because weight and cost can be reduced, and flexibility are excellent.

As an organic compound which forms the above organic semiconductor film, a condensed polycyclic aromatic compound having a specific structure is reviewed (for example, JP-A-2005-045266).

K. Yamane et al., Thin Solid Films, 516, 2758-61 (2008) discloses organic heterojunction ambipolar field effect transistors with asymmetric source and drain electrodes involving a naphthalene derivative of the formula 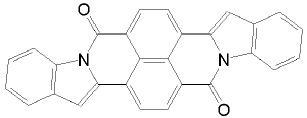 as an organic semiconducting compound.

### SUMMARY OF THE INVENTION

Organic semiconductor elements are generally required heat resistance because the semiconductor devices are incorporated into e.g. electronic devices to be used. That is, it is required to continuously exhibit sufficient semiconductor characteristics even in use under high temperature environment.

An object of the present invention is to provide an organic semiconductor element which exhibit desired semiconductor characteristics (for example, higher carrier mobility) and hardly causes deterioration of semiconductor characteristics even in a case of being exposed to a higher temperature environment. Another object of the present invention is to provide an organic semiconductor film suitable as an organic semiconductor layer in the organic semiconductor element and a manufacturing method thereof. Another object of the present invention is to provide a compound, a polymer, and a composition suitable as a constituent material of the organic semiconductor film.

To solve the above problems the present invention provides a compound of Formula (1) or (2) or a polymer having at least one structural unit of any of Formulae (9) and (10), 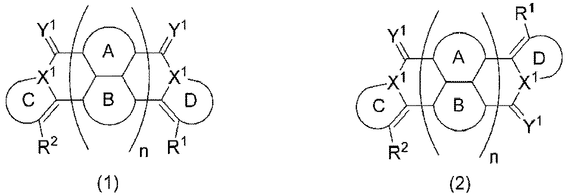 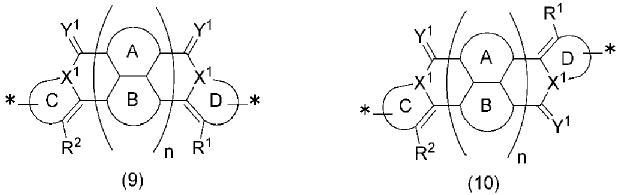 wherein
the fused ring structure formed of rings A and B is a structure of formula (4): 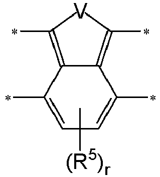 wherein
- R⁵: each is halogen or a group ^{∗}-L-T,
- V: is O, S, Se or NR^{d} wherein R^{d} is H or a substituent,
- r: is an integer of 0-2, and
- ^{∗}: is a bonding site.
- X¹: is N or CR^{a},
- rings C, D: each are a 5- or 6-membered aromatic or heteroaromatic ring, or a fused ring including the same,
- Y¹: is O or S,
- R^{a}, R^{b}, R^{c}: each are H or a substituent,
- n: is 1,
- ^{∗}: is a bonding site,
- R¹, R²: each are halogen or a group ^{∗}-L-T, wherein
- ^{∗}: is a bonding site,
- T: is H, halogen or cyano, and
- L: is a single bond, a divalent group of any of the formulae (L-1) to (L-25), or a divalent group formed by bonding two or more divalent groups of any of (L-1) to (L-25): 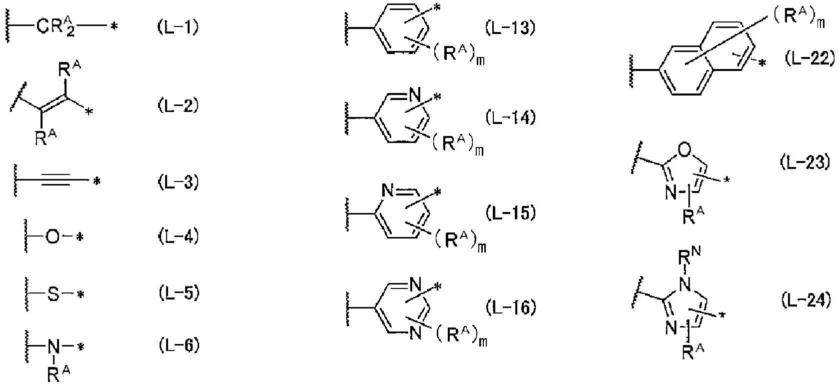 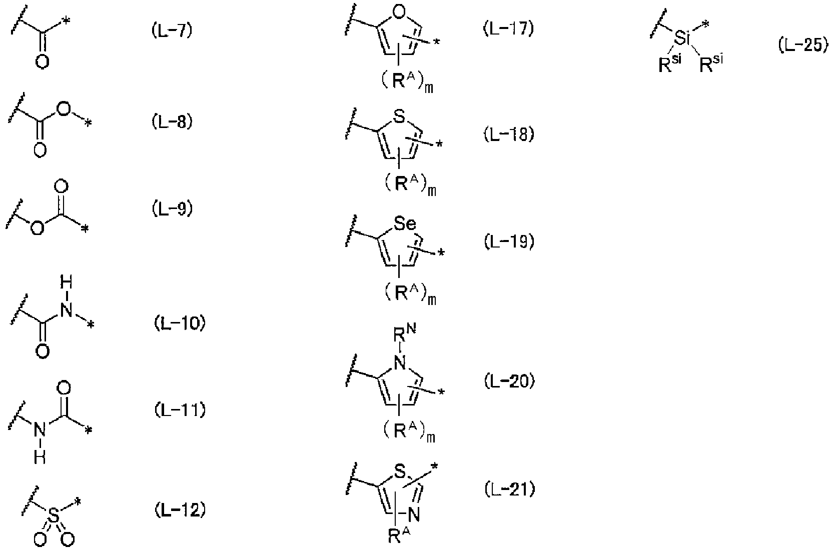
wherein
the wavy line portion is a bonding site to a ring structure of formula (1), (2), (9) or (10), or a bonding site to ^{∗} of a divalent group of any of formulae (L-1) to (L-25),
- ^{∗}: is a bonding site to T, or a bonding site to a wavy line portion of a divalent group of any of Formulae (L-1) to (L-25),
- R^{A}: is H or a substituent,
- R^{N}: is H or a substituent,
- R^{si}: is H, alkyl, alkenyl or alkynyl; and
- m: is an integer of 1-4 in formula (L-13), is an integer of 1-3 in formulae (L-14) and (L-15), is 1 or 2 in formulae (L-16) to (L-20) and is an integer of 1-6 in formula (L-22).

Also, the present invention provides an organic semiconductor element, comprising an organic semiconductor layer containing a compound of formula (1) and/or a compound of formula (2), or containing a polymer having at least one structural unit of any of formulae (9) and (10).

Further, the present invention provides an organic semiconductor composition comprising a compound of formula (1) and/or a compound of (2), or a polymer having at least one structural unit of any of formulae (9) and (10); a solvent; and optionally a binder.

Yet further, the present invention provides a method for manufacturing an organic semiconductor film, comprising coating a substrate with the above organic semiconductor composition to form a coating film; and drying the coating film.

Last, but not least, the present invention provides an organic semiconductor film comprising a compound of formula (1) and/or a compound of formula (2), or a polymer having at least one structural unit of any of formulae (9) and (10.

Preferred embodiments of the invention are as defined in the appended dependent claims and/or in the following detailed description. Hereinafter, the above aspects and embodiments of the invention will also be referred to as the present compound, the present organic semiconductor element, the present organic semiconductor composition, the present method, and the present organic semiconductor film, respectively.

In the present organic semiconductor element, desired semiconductor characteristics are exhibited, and semiconductor characteristics are hardly decreased even in a case of being exposed to a high temperature environment. In a case where the present organic semiconductor film is used as an organic semiconductor layer in an organic semiconductor element, it is possible to cause the obtained organic semiconductor element to exhibit desired semiconductor characteristics and to have characteristics in which the semiconductor characteristics hardly decrease even in a case of being exposed to a high temperature environment. By the present method, it is possible to obtain an organic semiconductor film having the excellent characteristics. The compound, the polymer, and the present organic semiconductor composition are appropriate as constituent materials of the organic semiconductor film.

### BRIEF DESCRIPTION OF THE DRAWINGS

Fig. 1 is a cross-sectional schematic view illustrating a bottom gate-bottom contact-type organic thin film transistor element which is an example of the present semiconductor element.
Fig. 2 is a cross-sectional schematic view illustrating a bottom gate-top contact-type organic thin film transistor element which is an example of the present semiconductor element.

### DESCRIPTION OF THE PREFERRED EMBODIMENTS

In the present specification, the following definitions apply

The numerical range expressed by using "to" means a range including numerical values described before and after "to" as a lower limit value and an upper limit value.

The expression of a compound includes the compound itself, a salt thereof, and an ion thereof. A portion of the structure may be changed without deteriorating the desired effect.

A compound which is not explicitly described as substituted or unsubstituted includes those having a random substituent without deteriorating the desired effect. The same is also applied to e.g. a substituent, a linking group and a ring structure(hereinafter, referred to as e.g. a substituent).

In a case where there are a plurality of e.g. substituents represented by a specific symbol, or in a case where a plurality of e.g. substituents are simultaneously defined, unless described otherwise, respective e.g. substituents may be identical to or different from each other. The same is also applied to the definition of the number of e.g. substituents. In a case where a plurality of e.g. substituents are near (particularly, adjacent to each other), unless described otherwise, the e.g. substituents may be connected to each other to form a ring.

In a case where a plurality of repeating units represented by the same chemical structure in the polymer are present, the respective repeating units present in the polymer may be identical to or different from each other. The same applies to each group forming the repeating unit.

The number of carbon atoms of the group is limited, the number of the carbon atoms of the group means the total number of carbon atoms including the substituent, unless described otherwise.

In the case where the group can form an acyclic skeleton and a cyclic skeleton, unless described otherwise, the group includes an acyclic skeleton group and a cyclic skeleton group. For example, alkyl includes straight chain alkyl, branched alkyl, and cyclic (cyclo) alkyl. In a case where the group can form a cyclic skeleton, the lower limit of the number of atoms of the group forming the cyclic skeleton is 3 or more and preferably 5 or more, regardless of the lower limit of the number of atoms specifically described for this group.

The preferable embodiment of the present invention is described below.

### [Organic semiconductor element]

In the present organic semiconductor element, the organic semiconductor layer is formed by using a compound having a specific structure and a polymer having a specific structure.

The present organic semiconductor element is not particularly limited, but is preferably used as a non-luminescent organic semiconductor device. The non-luminescent organic semiconductor device may be any device that is not intended to emit light, and examples thereof include an organic thin film transistor element that controls the amount of current or the amount of voltage, an organic photoelectric conversion element that converts light energy into electric power (such as an individual imaging device for light sensors or a solar cell for energy conversion), an organic thermoelectric conversion element for converting thermal energy into electric power, a gas sensor, an organic rectifying element, an organic inverter, and an information recording element. The non-luminous organic semiconductor device preferably causes the organic semiconductor film to function as an electronic element.

As a representative example of the organic semiconductor element, an organic thin film transistor element is described. An aspect in which the compound having a specific structure and a polymer having a specific structure constitute the organic semiconductor layer of the organic thin film transistor element is described, but the present invention is not limited to the aspect. That is, all of the organic semiconductor element in an aspect in which the organic semiconductor layer contains the compound having a specific structure and the polymer having a specific structure are included in the organic semiconductor element according to the embodiment of the present invention. The organic semiconductor layer having various elements can be formed by the method of forming the organic semiconductor layer in the organic thin film transistor element.

In the above description of the organic thin film transistor element, the improvement of carrier mobility is described, but the carrier mobility is a basic characteristics of the organic semiconductor. The organic semiconductor having high carrier mobility is not limited to the organic thin film transistor element, and can exhibit desired performances even in a case of being applied to each of the organic semiconductor elements.

### <Organic thin film transistor element>

The present organic thin film transistor element (hereinafter, also referred to as an organic TFT element) has an organic semiconductor film (hereinafter, also referred to as an organic semiconductor layer or a semiconductor active layer), and also has a source electrode, a drain electrode, and a gate electrode.

The present organic TFT element includes a gate electrode, an organic semiconductor layer, a gate insulating layer provided between the gate electrode and the organic semiconductor layer, and a source electrode and a drain electrode that are provided in contact with the organic semiconductor layer and are linked to each other via the organic semiconductor layer, on the substrate. In this organic TFT element, the organic semiconductor layer and the gate insulating layer are provided to be adjacent to each other.

The structure of the present organic thin film transistor element is not particularly limited, as long as the above respective layers are provided. For example, the organic TFT may have any structures of a bottom contact type (a bottom gate-bottom contact type and a top gate-bottom contact type) or a top contact type (a bottom gate-top contact-type and a top gate-top contact type). The present organic thin film transistor element is more preferably a bottom gate-bottom contact type or a bottom gate-top contact type (these are collectively referred to as a bottom gate type).

Hereinafter, an example of the present organic TFT element is described with reference to the drawings.

### -Bottom gate-bottom contact-type organic thin film transistor element-

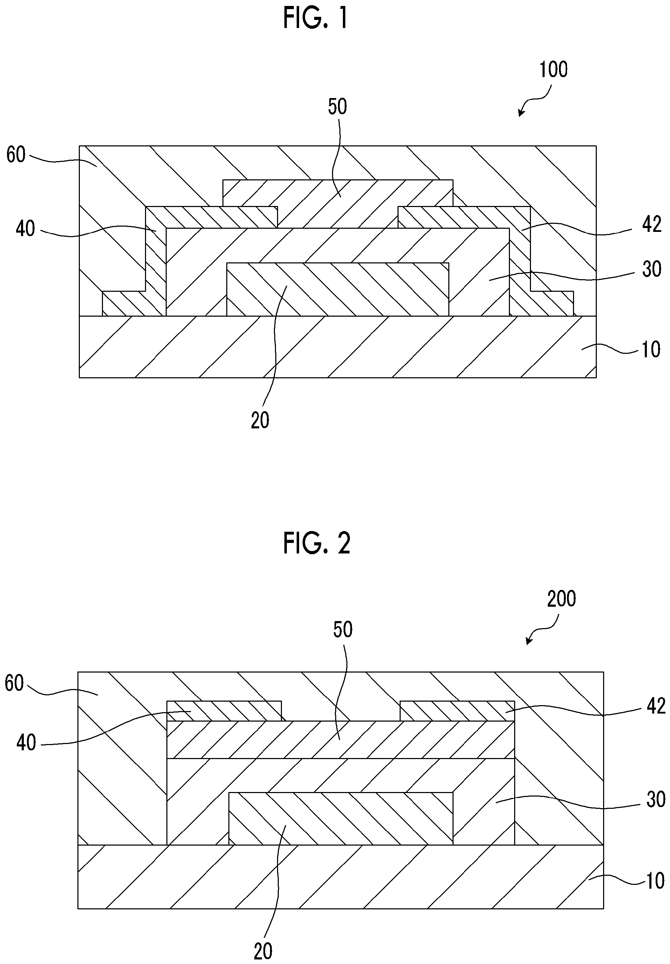

Fig. 1 is a cross-sectional schematic view of the bottom gate-bottom contact-type organic TFT element 100 which is an example of the present semiconductor element.

As illustrated in Fig. 1, the organic TFT element 100 has a substrate (base material) 10, a gate electrode 20, a gate insulating film 30, a source electrode 40, a drain electrode 42, an organic semiconductor film 50, and a sealing layer 60, in this order.

Hereinafter, a substrate (base material), a gate electrode, a gate insulating film, a source electrode, a drain electrode, an organic semiconductor film, a sealing layer, and a manufacturing method thereof are described above.

### (Substrate)

The substrate achieves a role of supporting, for example, a gate electrode, a source electrode and a drain electrode described below.

The types of the substrate are not particularly limited, and examples thereof include a plastic substrate, a silicon substrate, a glass substrate, or a ceramic substrate. Among these, in view of applicability to each device and costs, a glass substrate or a plastic substrate is preferable.

The thickness of the substrate is not particularly limited, and examples thereof is preferably ≤ 10 mm, more preferably ≤ 2 mm, and particularly preferably ≤ 1.5 mm. Meanwhile, the thickness is preferably ≥ 0.01 mm and more preferably ≥ 0.05 mm.

### (Gate electrode)

As the gate electrode, a well-known electrode that is used as a gate electrode of an organic TFT element may be used without particular limitation.

A material (electrode material) for forming the gate electrode is not particularly limited, and examples thereof include metal such as gold, silver, aluminum, copper, chromium, nickel, cobalt, titanium, platinum, magnesium, calcium, barium, and sodium, conductive oxide such as InO₂, SnO₂ and indium tin oxide (ITO) a conductive polymer such as polyaniline, polypyrrole, polythiophene, polyacetylene, and polydiacetylene, semiconductor such as silicon, germanium, and gallium arsenide, and a carbon material such as fullerene, carbon nanotube, and graphite. Among these, the above metal is preferable, and silver or aluminum is more preferable.

The thickness of the gate electrode is not particularly limited, but is preferably 20-200 nm.

The gate electrode may function as the substrate, and in this case, the above substrate may not be provided.

The method of forming the gate electrode is not particularly limited, and examples thereof include a method of performing vacuum deposition (hereinafter, simply referred to as vapor deposition) or sputtering on the electrode material on the substrate and a method of applying or printing an electrode forming composition that contains the electrode material. In the case of patterning the electrode, examples of the patterning method include a printing method such as inkjet printing, screen printing, offset printing, or toppan printing (flexographic printing), a photolithography method, and a mask vapor deposition method.

### (Gate insulating layer)

The gate insulating layer is not particularly limited, as long as the gate insulating layer is a layer having insulating properties, and may be a single layer or multiple layers.

The gate insulating layer is preferably formed of insulating materials, and examples of the insulating materials preferably include an organic polymer or inorganic oxide.

The organic polymer and the inorganic oxide are not particularly limited, as long as the organic polymer and the inorganic oxide have insulating properties. It is preferable to form a thin film, for example, a thin film having a thickness of ≤ 1 µm.

The organic polymer and the inorganic oxide may be used singly, two or more kinds thereof may be used in combination, or an organic polymer and inorganic oxide may be used in combination.

The organic polymer is not particularly limited, and examples thereof include polyvinyl phenol, polystyrene (PS), poly(meth)acrylate represented by polymethyl methacrylate, polyvinyl alcohol, polyvinyl chloride (PVC), polyvinylidene fluoride (PVDF), polytetrafluoroethylene (PTFE), a cyclic fluoroalkyl polymer represented by CYTOP (registered trademark), polycycloolefin, polyester, polyethersulfone, polyether ketone, polyimide, an epoxy resin, polyorganosiloxane represented by polydimethylsiloxane (PDMS), polysilsesquioxane, or butadiene rubber. In addition to the above, examples thereof include a thermosetting resin such as a phenol resin, a novolak resin, a cinnamate resin, an acrylic resin, or a polyparaxylylene resin.

The organic polymer may be used in combination with a compound having a reactive substituent such as an alkoxysilyl group, a vinyl group, an acryloyloxy group, an epoxy group, or a methylol group.

In a case where the gate insulating layer is formed with an organic polymer, it is preferable to crosslinking and hardening the organic polymer for the purpose of increasing solvent resistance or insulation resistance of the gate insulating layer. The crosslinking is preferably performed by using light, heat, or both, so as to generate acid or radical.

In a case where crosslinking is performed with a radical, as a radical generating agent that generates radicals by light or heat, for example, thermal polymerization initiators (HI) and photopolymerization initiators (H2) disclosed in paragraphs [0182]-[0186] of JP-A-2013-214649, photoradical generating agents disclosed in paragraphs [0046]-[0051] of JP-A-2011-186069, or photoradical polymerization initiators disclosed in paragraphs [0042]-[0056] of JP-A-2010-285518 can be suitably used.

The "compound (G) having number-average molecular weight (Mn) of 140-5,000, having crosslinking functional groups, and not having a fluorine atom" disclosed in paragraphs [0167]-[0177] of JP-A-2013-214649 is preferably used.

In the case of crosslinking with an acid, examples of the photoacid generator that generates acid by light include photo cationic polymerization initiators disclosed in paragraphs [0033]-[0034] of JP-A-2010-285518, acid generators disclosed in paragraphs [0120]-[0136] of JP-A-2012-163946, particularly sulfonium salts, and iodonium salts may be preferably used.

As a thermal acid generator (catalyst) that generates acid by heat, for example, thermal cation polymerization initiators and particularly onium salts disclosed in paragraphs [0035]-[0038] of JP-A-2010-285518, catalysts disclosed in paragraphs [0034]-[0035] of JP-A-2005-354012, particularly, sulfonic acids and sulfonic acid amine salts preferably can be used.

Crosslinking agents, particularly difunctional or higher epoxy compounds and oxetane compounds disclosed in paragraphs [0032]-[0033] of JP-A-2005-354012, crosslinking agents, particularly compounds, each of which has two or more crosslinking groups and in which at least one of these crosslinking groups is a methylol group or a NH group, disclosed in paragraphs [0046]-[0062] of JP-A-2006-303465, and compounds, each of which has two or more of hydroxymethyl groups or alkoxymethyl groups in a molecule, disclosed in paragraphs [0137]-[0145] of JP-A-2012-163946, are preferably used.

The method forming a gate insulating layer with an organic polymer is not particularly limited, and examples thereof include a method of applying the coating solution containing the organic polymer, and curing the coating solution, if necessary.

A solvent used in the coating solution is not particularly limited, as long as the solvent does not dissolve but can disperse the organic polymer, and can be appropriately selected from solvents generally used according to e.g. the kinds of the organic polymer.

The coating method is not particularly limited, and examples thereof include the above printing methods. Among these, a wet coating method such as a micro gravure coating method, a dip coating method, screen coating printing, a die coating method, or a spin coating method is preferable.

The coating conditions are not particularly limited, and can be appropriately set.

The curing method and conditions are not particularly limited, as long as the organic polymer can be crosslinked by the method and conditions, and can be appropriately set, for example, according to the crosslinking method (radical or acid), and also the kinds of a photoacid generator or thermal acid generator used.

The inorganic oxide is not particularly limited, and examples thereof include oxide such as silicon oxide, silicon nitride (SiN_{Y}), hafnium oxide, titanium oxide, tantalum oxide, aluminum oxide, niobium oxide, zirconium oxide, copper oxide, and nickel oxide, a compound having a perovskite structure such as SrTiO₃, CaTiO₃, BaTiO₃, MgTiO₃, and SrNb₂O₆, and composite oxide or mixture of these.

Here, in addition to silicon oxide (SiOx), the silicon oxide includes Boron Phosphorus Silicon Glass (BPSG), Phosphorus Silicon Glass (PSG), borosilicate glass (BSG), arsenic silicate glass (AsSG), lead silicate glass (PbSG), silicon oxynitride (SiON), spin-on-glass (SOG), a low dielectric constant SiO₂-based material (for example, polyaryl ether, a cycloperfluorocarbon polymer, benzocyclobutene, a cyclic fluororesin, polytetrafluoroethylene, fluoroaryl ether, fluorinated polyimide, amorphous carbon, organic SOG).

The method of forming a gate insulating layer with inorganic oxide is not particularly limited, and a vacuum film formation method such as a vacuum evaporation method, a sputtering method, an ion plating method, or a chemical vapor deposition (CVD) method can be used. Plasma using any gas, an ion gun or a radical gun may be also used during film formation.

A gate insulating layer can be formed by causing a precursor corresponding to each of the metal oxide, specifically, metal halides such as chlorides and bromides, metal alkoxide, and metal hydroxide, to react with an acid such as hydrochloric acid, sulfuric acid, and nitric acid and a base such as sodium hydroxide or potassium hydroxide in alcohol or water so as to perform hydrolysis. In a case where such a solution-based process is used, a wet-coating method can be used.

In a case where the gate insulating layer is formed with inorganic oxide, in addition to the above method, it is possible to use a method of combining any one of a lift-off method, a sol-gel method, an electrodeposition method, and a shadow mask method, with a patterning method, if necessary.

A surface treatment such as a corona treatment, a plasma treatment, an ultraviolet (UV)/ozone treatment may be performed on the gate insulating layer. In this case, it is preferable that the surface roughness is not roughened by each treatment. For example, it is preferable that arithmetic mean roughness Ra or root mean square roughness R_{q} (all are JIS B0601: 2013) of the gate insulating layer surface after the treatment is ≤ 0.5 nm.

The thickness of the gate insulating film is not particularly limited but is preferably 100-1,000 nm.

### (Source electrode and drain electrode)

In the present organic TFT element, the source electrode is an electrode in which a current flows from the outside through wire. The drain electrode is an electrode in which a current is sent to the outside through wire.

As a material for forming the source electrode and the drain electrode, the same materials as the electrode material for forming the above gate electrode may be used. Among these, metal is preferable, and silver is more preferable.

The thicknesses of the source electrode and the drain electrode are not particularly limited, but each are preferably ≥ 1 nm and particularly preferably ≥ 10 nm. The thickness is preferably ≤ 500 nm and particularly preferably ≤ 300 nm.

The distance (gate length) between the source electrode and the drain electrode may be appropriately determined, but for example, the distance is preferably ≤ 200 µm and particularly preferably ≤ 100 µm. The gate width may be appropriately determined, but for example, the gate width is preferably ≤ 5,000 µm and particularly preferably ≤ 1,000 µm.

The method of forming the source electrode and the drain electrode is not particularly limited, and examples thereof include a method of performing vacuum deposition or sputtering on the electrode material on the substrate on which the gate electrode and the gate insulating film are formed or a method of applying or printing the electrode forming composition. In the case of patterning, the patterning method is the same as the method of the gate electrode described above.

### (Organic semiconductor layer (film))

In an embodiment of the organic TFT element (hereinafter, also referred to as a "first embodiment"), the organic semiconductor layer contains a compound of Formula (1) and/or a compound of Formula (2). The compound of Formula (1) is referred to as a cis isomer, and the compound of Formula (2) is referred to as a trans isomer:

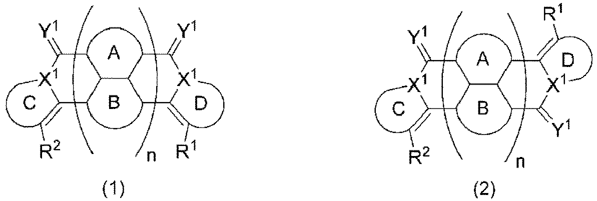

In Formulae (1) and (2), the fused ring structure formed of rings A and B is a structure of formula (4): 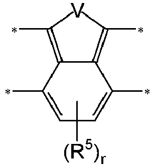 wherein R⁵ each is halogen or a group ^{∗}-L-T as defined below, V is O, S, Se or NR^{d} wherein R^{d} is H or a substituent, r is an integer of 0-2, and ^{∗} is a bonding site, X¹ is N or CR^{a}, rings C, D each are a 5- or 6-membered aromatic or heteroaromatic ring, or a fused ring including the same. The fused ring is preferably a two-ring structure or a three-ring structure, more preferably a two-ring structure. R^{a} is H or a substituent. X¹ is preferably N.

Y¹ is O or S, preferably O.

The substituents R^{a}, R^{b}, and R^{c} are not particularly limited, and include a group ^{∗}-L-T, and examples thereof are halogen (F, Cl, Br and I are preferable, F and Cl are more preferable, and F is particularly preferable). Among these, as the substituents R^{a}, R^{b}, and R^{c}, alkyl (an C₁₋₃₅-alkyl is preferable, and C₁₋₃₅-alkyl is more preferable), alkenylene (the number of carbon atoms thereof is preferably 2-30), alkynyl (the number of carbon atoms thereof is preferably 2-30), an aromatic hydrocarbon group (the number of carbon atoms thereof is preferably 6-30), an aromatic heterocyclic ring group (a 5-membered to 7-membered ring is preferable; a ring-constituting atom preferably includes at least one of O, N, S and Se), or halogen (F, Cl, Br or I are preferable, F and Cl are more preferable, and F is particularly preferable.) is preferable.

The alkenyl group, the alkenyl group, and the alkynyl group employed as R^{a}, R^{b}, and R^{c} may include at least one of -O-, -S-, or -NR^{X1}- in a carbon chain or at a terminal of the carbon chain. R^{X1} is H or a substituent. The number of -O-, -S-, and -NR^{X1}- included in the carbon chain or at a terminal of a carbon chain is preferably an integer of 1-5, more preferably 1-3, and even more preferably 1 in total.

The substituent that can be employed as R^{X1} is not particularly limited, and examples thereof include alkyl (preferably C₁₋₁₀-alkyl), a halogen atom (preferably F, Cl, Br or I), or an aromatic hydrocarbon group (preferably an aromatic hydrocarbon group having 6-20 carbon atoms). R^{X1} is preferably H or alkyl and more preferably alkyl.

R¹ and R² each are halogen (preferably F, Cl, Br or I), or a group ^{∗}-L-T.
in the group ^{∗}-L-T as R¹ and R², L is a single bond, a divalent group of any one of Formulae (L-1) to (L-25), or a divalent group formed by bonding two or more divalent groups represented by any of (L-1) to (L-25). The two or more bonded divalent groups may be identical to as or different from each other.

T is H, halogen (preferably F, Cl, Br or I) or cyano.

^{∗} is a bonding site to the ring C or D presented in Formulae (1) and (2).

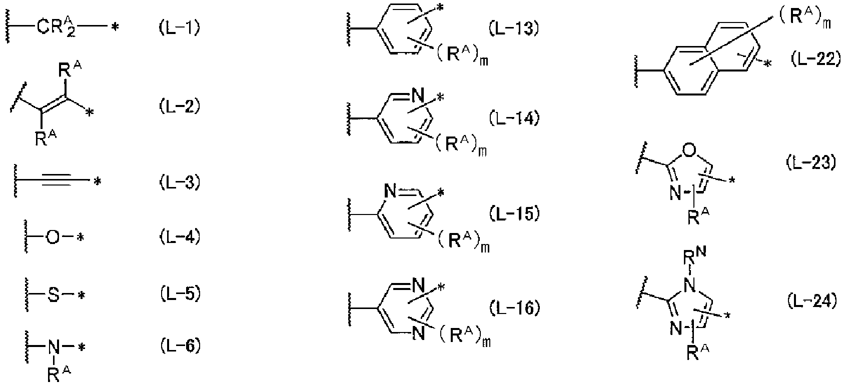

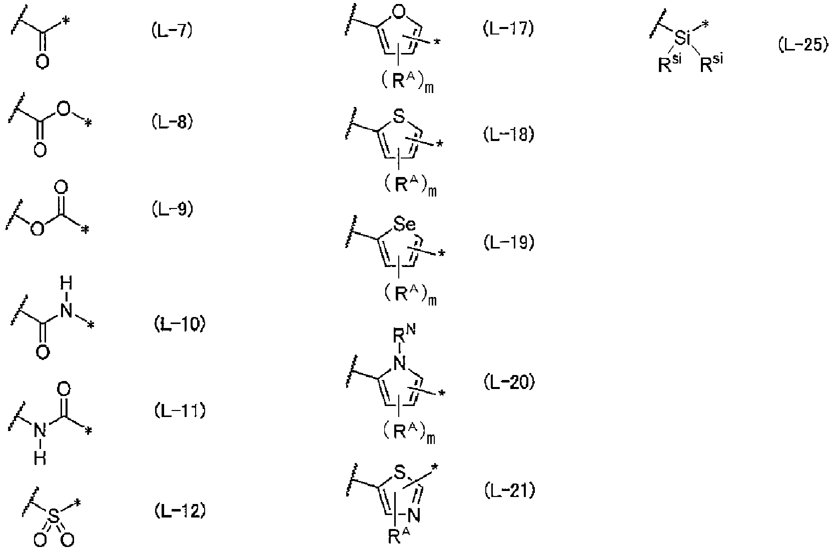

In Formulae (L-1) to (L-25), the wavy line portion is a bonding site to a ring structure of Formula (1) or (2), or a bonding site to ^{∗} of a divalent group of any one of Formulae (L-1) to (L-25).
^{∗} is a bonding site to T, or a bonding site to a wavy line portion of a divalent group of any one of Formulae (L-1) to (L-25).

In Formulae (L-1), (L-2), (L-6), and (L-13) to (L-24), R^{A} is H or a substituent.

In Formula (L-13), m is an integer of 1-4, and m in Formulae (L-14) and (L-15) is an integer of 1-3, and m in Formulae (L-16) to (L-20) is 1-2, and m in Formula (L-22) is an integer of 1-6.

In Formulae (L-20) and (L-24), R^{N} is H or a substituent.

In Formula (L-25), R^{si} is H, alkyl, alkenyl or alkynyl.

The divalent groups of Formulae (L-17) to (L-21), (L-23), and (L-24) are preferably structures of Formulae (L-17A) to (L-21A), (L-23A), and (L-24A), respectively. R^{A}, R^{N}, m, and ^{∗} in Formula (L-17A) to (L-21A), (L-23A), and (L-24A) are the same as R^{A}, R^{N}, m, and ^{∗} in Formulae (L-17) to (L-21), (L-23), and (L-24):

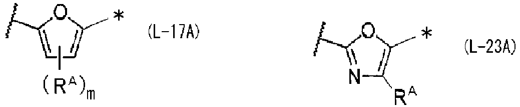

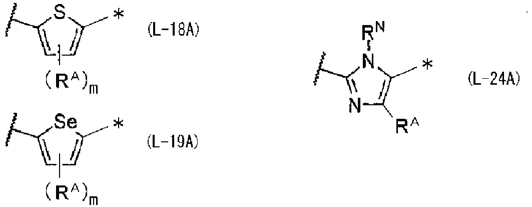

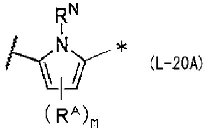

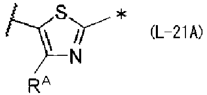

L is a divalent group selected from Formulae (L-1), (L-2), (L-3), (L-4), (L-13), (L-17), and (L-18), or a group obtained by bonding two or more divalent groups selected from Formulae (L-1), (L-2), (L-3), (L-4), (L-13), (L-17), and (L-18).

The molecular weight of -L- is preferably ≤ 1,000, more preferably ≤ 600, and even more preferably ≤ 300.

The substituent that may be employed as R^{A} and R^{N} is not particularly limited, and, for example, a group selected from alkyl, alkenyl, alkynyl, an aromatic hydrocarbon group, an aromatic heterocyclic group, and halogen is preferable, and preferable embodiments thereof are respectively the same as those defined for R^{a} to R^{c}.

The preferable embodimentsof the alkyl, alkenyl and alkynyl that can be employed as R^{si} are respectively the same as those defined for R^{a} to R^{d}.

The group ^{∗}-L-T is preferably alkyl, alkenyl or alkynyl, the preferable embodimentsof the alkyl, alkenyl or alkynyl are respectively the same as those defined for R^{a} to R^{c}.

In Formulae (1) and (2), n is 1 or 2, and is preferably 1.

With respect to the organic TFT element, in a case where the organic semiconductor layer includes a compound of Formula (1) and/or a compound of Formula (2), both of the desired carrier mobility and heat resistance can be realized. The reason thereof is not clear, but it is thought that the effect largely depends on the action of a mother nucleus (the fused polycyclic structure provided in each formula) of each of the above compounds. Since N atoms are appropriately arranged in the mother nuclei and the asymmetric fused ring structure is formed in the minor axis direction, it is assumed that the intermolecular interaction between adjacent mother nuclei increases due to the dipole moment and the overlapping of the orbits increases to improve carrier mobility, and the rate of change in crystal structure is suppressed in a case of heating by increasing the intermolecular interaction between the adjacent mother nuclei to improve the heat resistance.

In Formula (4), R³ to R⁵ each are halogen or a group ^{∗}-L-T.

V is NR^{d}, O, S or Se, preferably S or Se, and more preferably S.

R^{d} is H or a substituent. The substituents in R^{d} preferably include the embodiments of the substituent in R^{a} to R^{c}.
p, q, and r each are an integer of 0-2, preferably 0 or 2, and more preferably 0.
^{∗} is a bonding site to a carbon atom to which Y¹ and X¹ in Formulae (1) and (2) are bonded, a carbon atom to which X¹ and R¹C are bonded, and a carbon atom to which X¹ and R²C are bonded.

In the fused ring structure of Formula (4), the ring on the upper side in the plane of the drawing may be the ring A and the ring on the lower side may be the ring B, or the reverse aspect thereof may be possible. That is, depending on the combination of the positions of the ring A and the ring B, the compound of Formula (1) can employ a cis isomer.

In Formulae (1) and (2), the rings C and D are preferably fused ring structures of Formula (5) or (6) and more preferably fused ring structures of Formula (5):

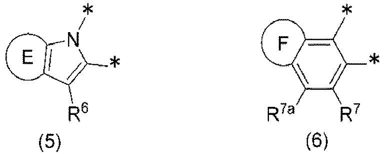

In Formulae (5) and (6), rings E and F each are a 5- or 6-membered aromatic or heteroaromatic ring, or a fused ring including a 5- or 6-membered aromatic or heteroaromatic ring. The embodiment of the fused ring including a 5- or 6-membered aromatic or heteroaromatic ring is preferably a two-ring structure. The rings E and F are preferably single ring structures.
R⁶, R⁷, and R^{7a} each are H, halogen or a group ^{∗}-L-T.
^{∗} represents a bonding site.

The fused ring structure of Formula (5) is preferably a fused ring structure of Formula (7) or (8) and more preferably a fused ring structure of Formula (8):

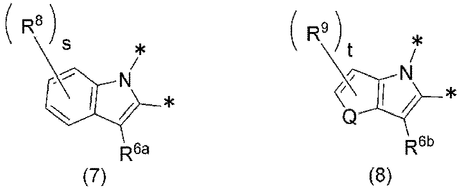

In Formulae (7) and (8), R^{6a} and R^{6b} each are H, halogen (preferably F, Cl, Br or I), or a group ^{∗}-L-T.
R⁸ and R⁹ each are halogen (preferably F, Cl, Br or I) or a group ^{∗}-L-T.
s is an integer of 0-4, preferably 1 or 2, and more preferably 1. t is an integer of 0-2 and preferably 1.
Q is a chalcogen atom. Q is preferably O or S and is more preferably S.
^{∗} represents a bonding site.

Specific preferable examples of the compound of Formulae (1) and (2) are provided below, but the present invention is not limited to these embodiments. In the following compounds, TMS represents trimethylsilyl.

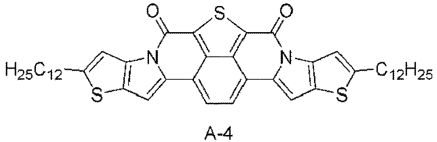

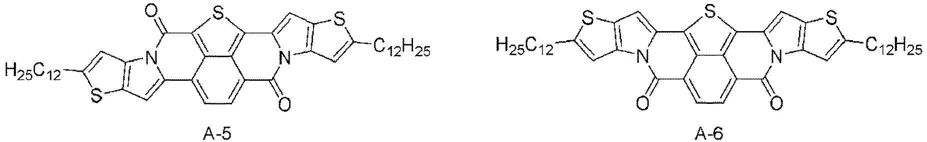

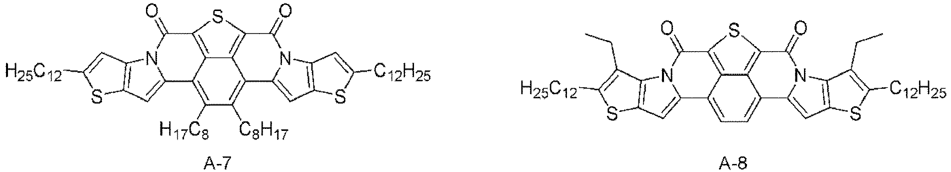

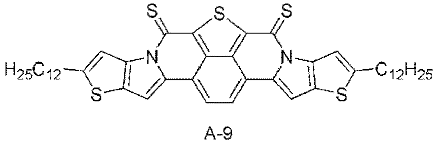

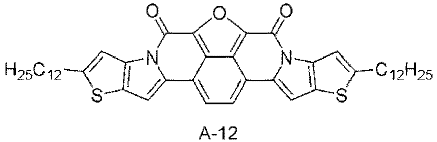

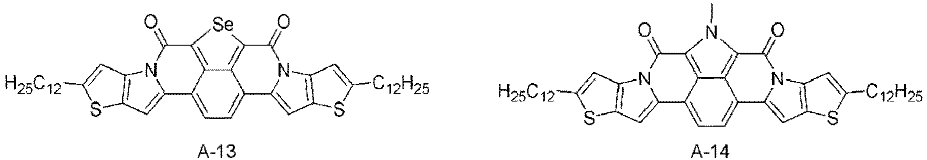

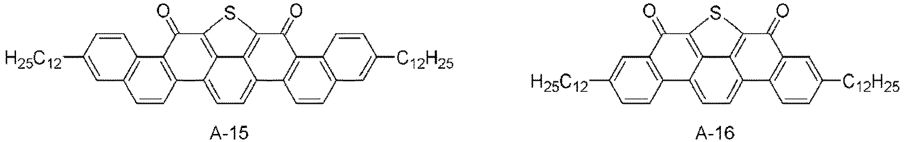

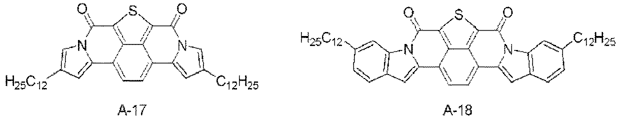

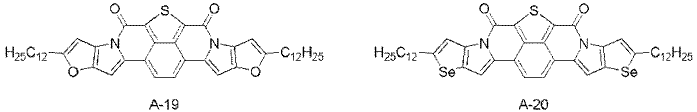

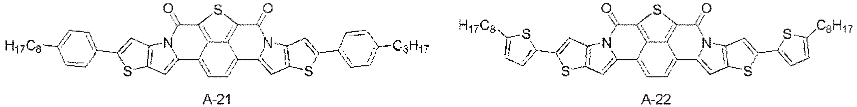

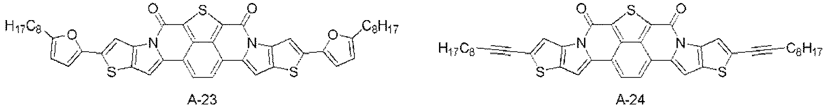

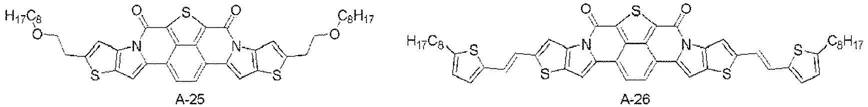

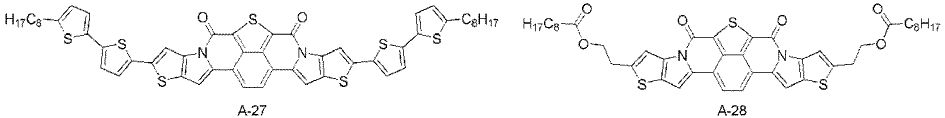

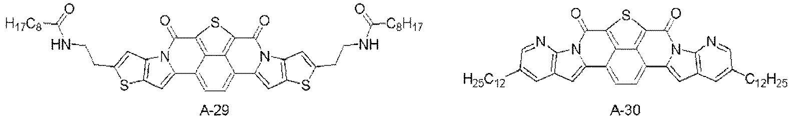

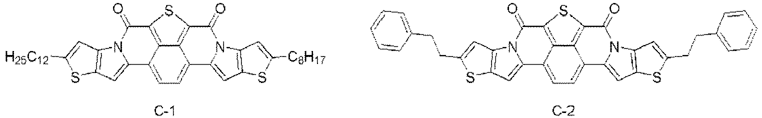

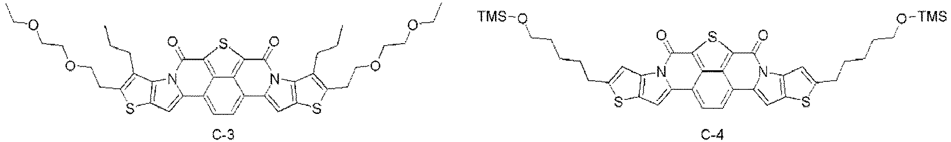

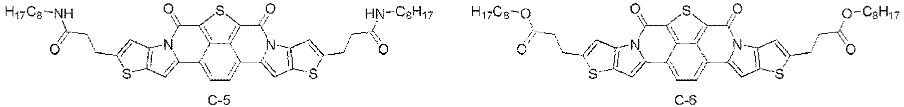

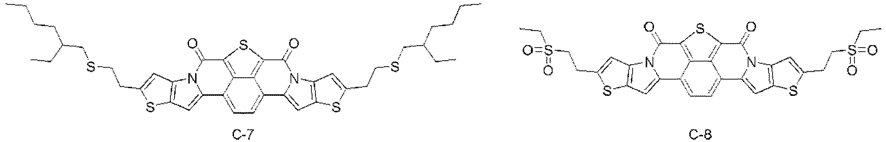

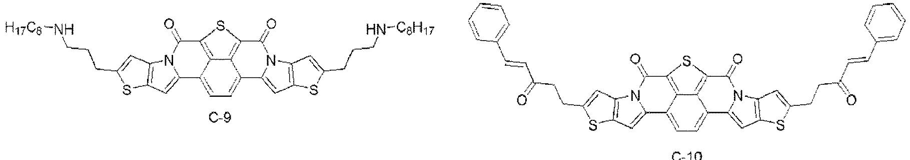

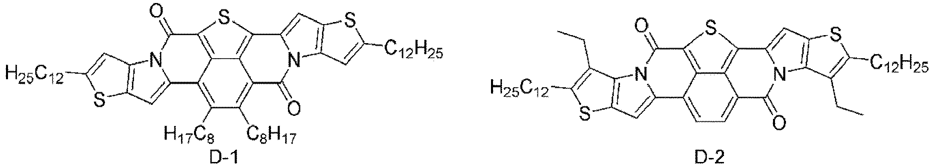

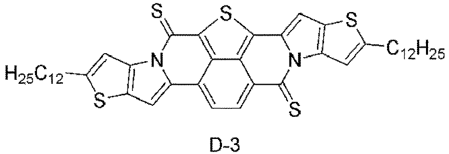

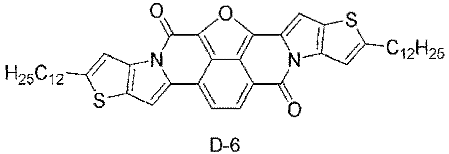

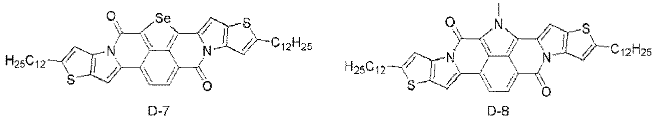

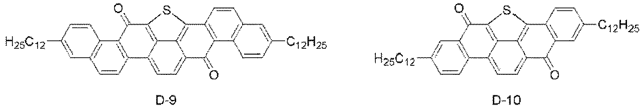

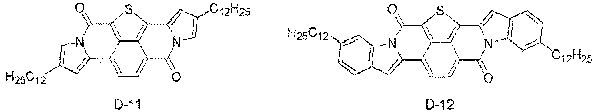

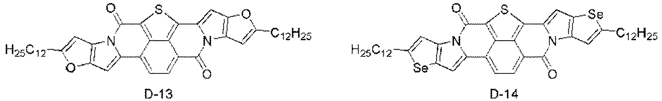

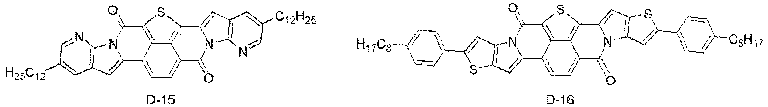

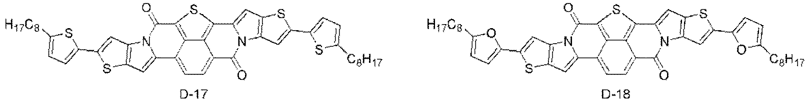

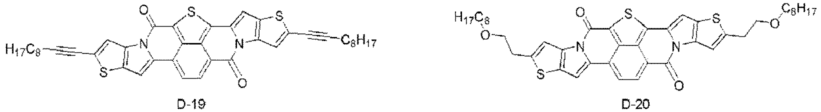

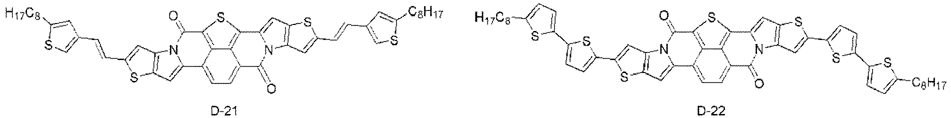

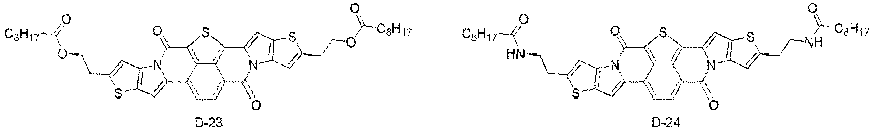

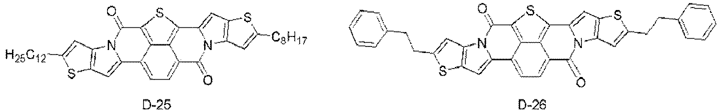

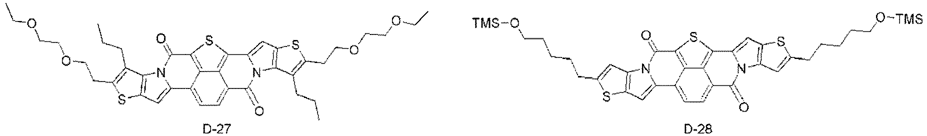

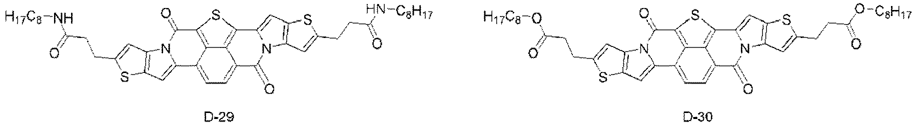

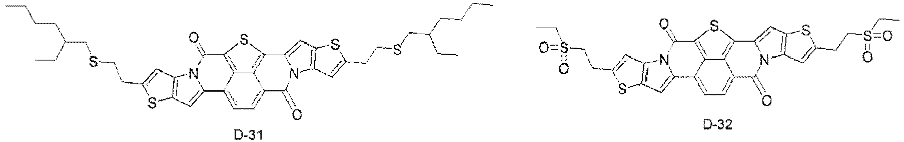

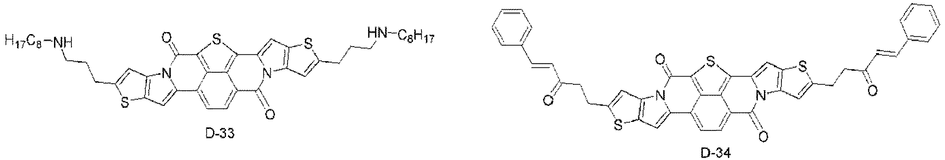

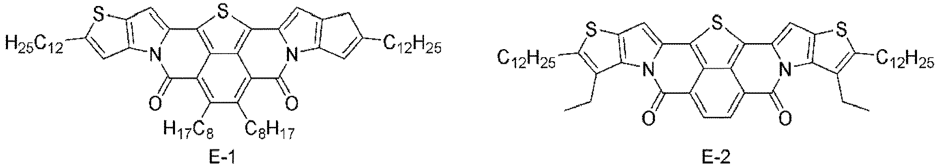

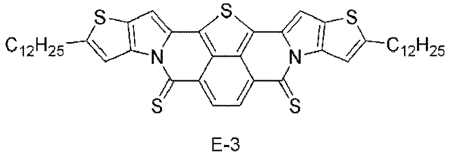

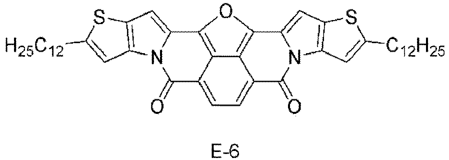

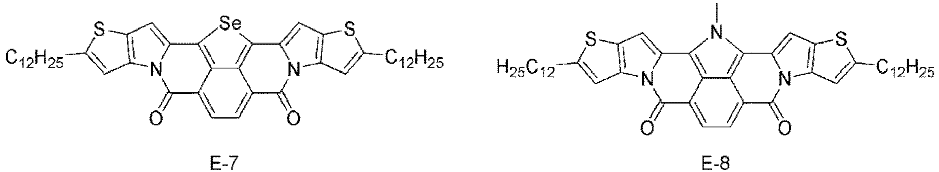

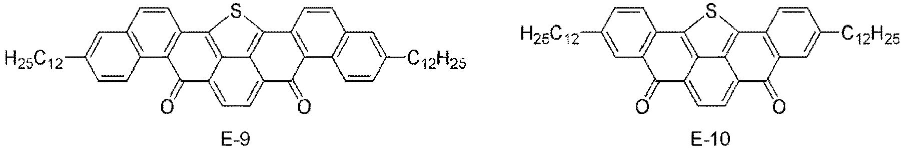

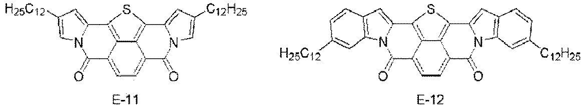

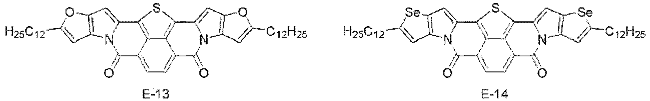

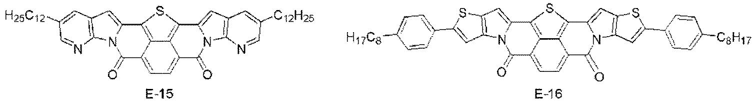

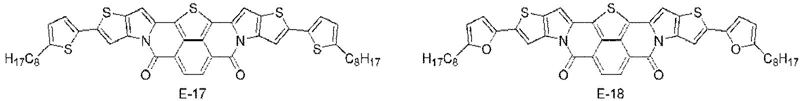

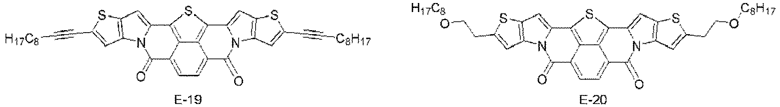

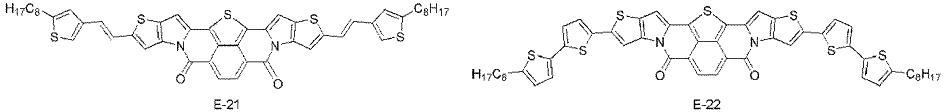

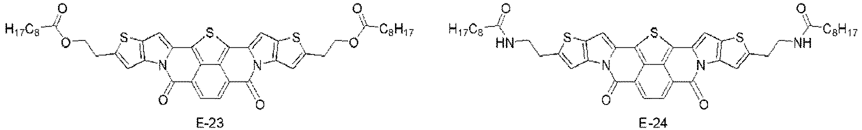

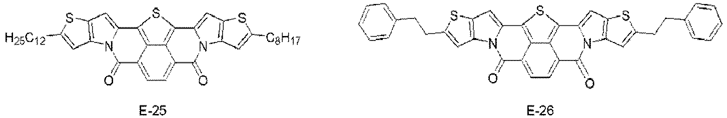

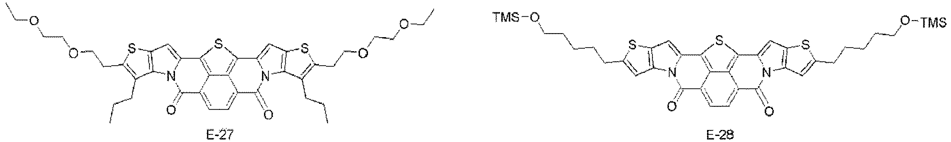

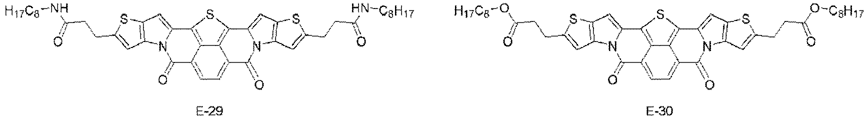

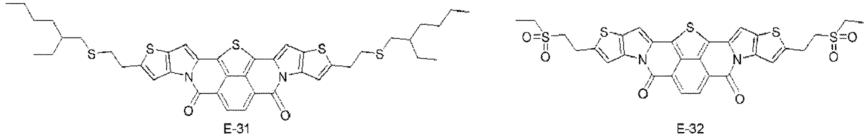

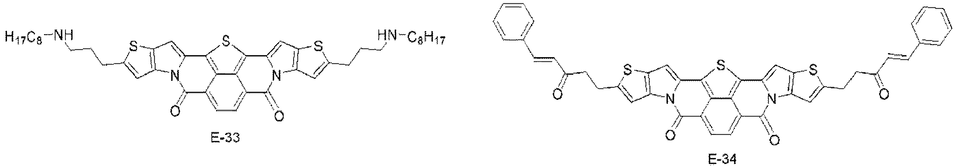

In another embodiment of the organic TFT element (hereinafter, also referred to as a "second embodiment"), the organic semiconductor layer contains a polymer having at least one structural unit of any of Formulae (9) and (10). The "polymer" in the second embodiment is used in a meaning of including an oligomer (for example, an oligomer having 2-10 repeating units). That is, in the present embodiment, the "polymer" is meant to include all compounds having two or more structural units of any of Formulae (9) and (10).

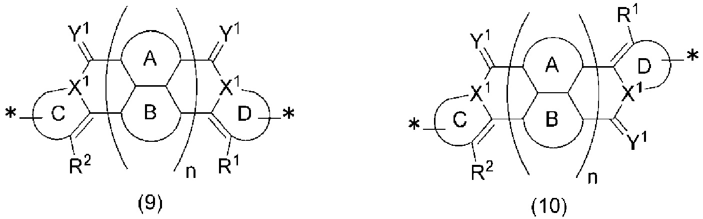

X¹, Y¹, the rings A to D, R¹, R², and n are respectively the same as X¹, Y¹, the rings A to D, R¹, R², and n in Formulae (1) and (2), and preferable embodiments thereof are also the same. Here, with respect to s and t in Formulae (7) and (8), relating to the rings C and D, s is preferably an integer of 0-3 and more preferably 0, and t is preferably 0. ^{∗} is a bonding site to be combined in the polymer.

With respect to the organic TFT element, in a case where the organic semiconductor layer includes a polymer having at least one structural unit of any of Formulae (9) and (10), both of the desired carrier mobility and heat resistance can be realized. The reason thereof is not clear, but it is thought that the effect largely depends on the action of a mother nucleus (the fused polycyclic structure provided in each formula) of each of the above structural units. That is, since N atoms are appropriately arranged in the mother nuclei and the asymmetric fused ring structure is formed in the minor axis direction, it is assumed that the intermolecular interaction between adjacent mother nuclei increases due to the dipole moment and the overlapping of the orbits increases to improve carrier mobility, and the rate of change in crystal structure is suppressed in a case of heating by increasing the intermolecular interaction between the adjacent mother nuclei to improve the heat resistance. This effect is more prominent in a case where the mother nucleus has a structure of being conjugated and linked in the main chain direction.

The polymer having at least one kind of the structural unit of Formulae (9) and (10) preferably has a structure of Formula (G).

^{∗}-Ar¹-(Vr)ₚ₃-Ar²-^{∗} (G)

In Formula (G), Ar¹ and Ar² each are a single bond, vinylene, ethynylene, arylene or heteroarylene, or is a divalent group formed by linking two or more groups selected from vinylene, ethynylene, arylene and heteroarylene.

The aryl group that can be included in Ar¹ and Ar² preferably has 6-30 carbon atoms, more preferably 6-20 carbon atoms, and even more preferably 6-15 carbon atoms. Preferable specific examples of this arylene group include phenylene and naphthylene, and phenylene is preferable.

The heterocyclic arylene group that can be included in Ar¹ and Ar² is preferably a 5- or 6-membered aromatic heterocyclic ring, or a fused heterocyclic ring including a 5- or 6-membered aromatic heterocyclic ring. The above heteroarylene group also includes an embodiment in which these aromatic heterocyclic ring or a fused heterocyclic ring has a substituent.

Examples of the 5-membered aromatic heterocyclic ring and the fused heterocyclic ring including a 5-membered aromatic heterocyclic ring include pyrrole, imidazole, pyrazole, oxazole, thiazole, isoxazole, isothiazole, triazole, oxadiazole, thiadiazole, furan, thiophene, benzimidazole, benzoxazole, benzothiazole and indazole.

Examples of the 6-membered aromatic heterocyclic ring and the fused heterocyclic ring including a 6-membered aromatic heterocyclic ring include pyridine, pyrimidine, pyrazine, pyridazine, triazine, quinoline, isoquinoline, quinoxaline, phthalazine, cinnoline and quinazoline.

In a case where Ar¹ and Ar² are divalent groups formed by linking two or more groups selected from vinylene, ethynylene, arylene and heteroarylene, the molecular weight of Ar¹ and Ar² is preferably 48-1,000 and more preferably 48-600.

Ar¹ and Ar² are more preferably a single bond or a divalent group of Formula (Ar-1) or (Ar-2):

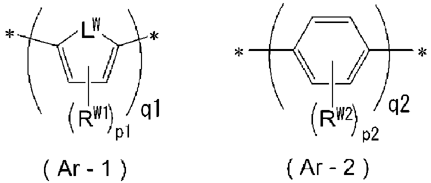

In Formula (Ar-1), R^{W1} and R^{W2} each are alkyl. p1 is an integer of 0-2 and is preferably 0 or 1. p2 is an integer of 0-4, preferably of 0-3, and more preferably of 0-2.

Preferable embodiments of the alkyl group that can be employed as R^{W1} and R^{W2} are the same as those that can be employed as R^{X1}.

L^{W} is a chalcogen atom, preferably O, S or Se, and more preferably S.

q1 and q2 are is an integer of 1-4, preferably 1 or 2, and more preferably 1.

In Formula (G), Vr is a divalent conjugated group having 2-40 carbon atoms. Vr is preferably a structure selected from Formulae (V_{D}-1) to (V_{D}-16), and (V_{A}-1) to (V_{A}-11).
p3 is an integer of 1-6, preferably 1 or 2, and more preferably 1.

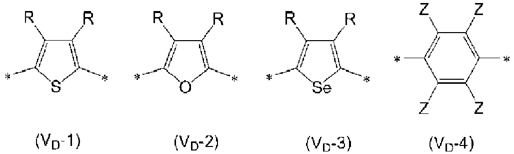

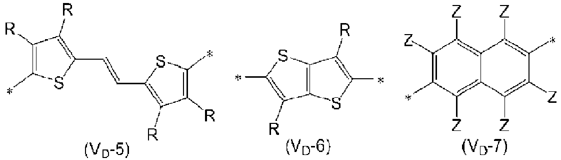

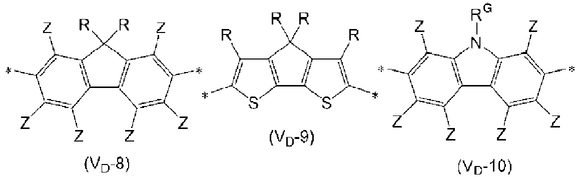

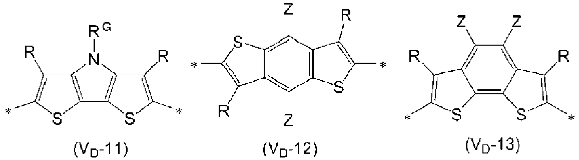

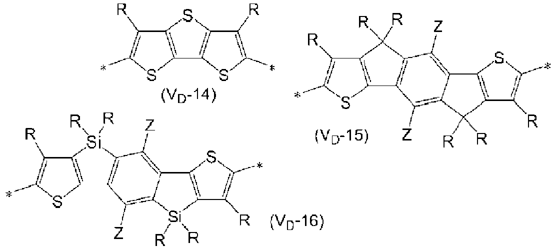

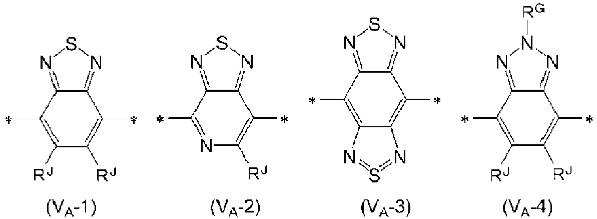

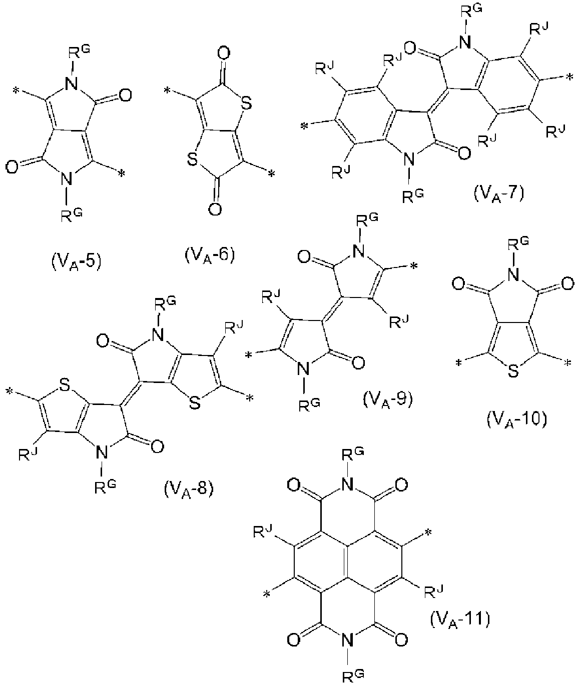

In each of the formulae, ^{∗} indicates a bonding site.

R and Z each are H, halogen (preferably F, Cl, Br or I) or alkyl. Preferable embodiments of the alkyl group that can be employed as R and Z are the same as those that can be employed as R^{a} to R^{c}.

R^{G} is alkyl. Preferable embodiments of the alkyl group are the same as those that can be employed as R^{a} to R^{c}.

R^{J} is H, alkyl, cyano or halogen. Preferable embodiments of the alkyl group and a halogen atom that can be employed as R^{J} are the same as those that can be employed as Z.

Vr is preferably a divalent group of any of Formulae (V_{D}-1) to (V_{D}-16).

Since the above polymer has a structure of Formula (G), a bias of electron density occurs in the main chain, the bias increases the interaction between the main chains to increase the overlapping of the orbits, and thus improvement of carrier mobility and improvement of heat resistance can be achieved.

The polymer preferably has alternately a structural unit of any of Formulae (9) and (10) and a structure of Formula (G). This structure is preferable, because the polymer can be conjugated in the main chain direction, and the bias of ionization potential in the main chain can be reduced.

The weight-average molecular weight of the polymer having at least one kind of the structural unit of Formulae (9) and (10) is 1,000-500,000 and more preferably 1,000-300,000.

In the present invention, the weight-average molecular weight and the number-average molecular weight are measured by a gel permeation chromatography (GPC) method and are calculated in terms of standard polystyrene. Specifically, for example, in GPC, HLC-8121 GPC (manufactured by Tosoh Corporation), is used, two units of TSKgel GMHHR-H (20) HT (manufactured by Tosoh Corporation, 7.8 mm ID × 30 cm) are used as and 1,2,4-trichlorobenzene is used as an eluent. As the conditions, a sample concentration of 0.02 mass%, a flow rate of 1.0 mL/min, a sample injection amount of 300 µL, and a measurement temperature of 160°C are set, and an infrared (IR) detector is used, so as to perform the GPC. The calibration curve is manufactured by using 12 samples of "Standard sample TSK standard, polystyrene": "F-128", "F-80", "F-40", "F-20", "F-10", "F-4", "F-2", "F-1", "A-5000", "A-2500", "A-1000", and "A-500".

The content of the structural unit of Formulae (9) and (10) in the polymer having at least one such structural unit is preferably 10-100 mass%, more preferably 30-90 mass%, and even more preferable 50-80 mass% in total.

The terminal structure of the polymer having at least one structural unit of any of Formulae (9) and (10) is not particularly limited and do not uniformly determined, according to the presence or absence of other repeating units, the type of base material used in the synthesis, or the types of the quenching agent during synthesis (reaction stopping agent). Examples of the structure of the terminal include H, OH, halogen, an ethylenically unsaturated group, alkyl, and an aromatic heterocyclic ring group (preferably a thiophene ring), or an aromatic hydrocarbon group (preferably a benzene ring).

The method of synthesizing at least one structural unit of any of Formulae (9) and (10) is not particularly limited, and the synthesis can be performed with reference to a well-known method. For example, synthesis can be performed by synthesizing precursor compounds that can derive a group derived from Formula (9) and each group of Formula (G), and performing cross-coupling reaction such as Suzuki coupling reaction or Stille coupling reaction on the respective precursors. In the synthesis of the polymer of the present invention, for example, publications of JP-A-2010-527327, JP-A-2007-516315, JP-A-2014-515043, JP-A-2014-507488, JP-A-2011-501451, JP-A-2010-018790, WO 2012/174561, JP-A-2011-514399 and JP-A-2011-514913 can be referred to.

Specific examples of the polymer having at least one structural unit of Formulae (9) and (10) are provided below, but the present invention is not limited to these embodiments. In the following polymers, a mixture of a cis isomer and a trans isomer is described using the structure of the trans isomer for convenience of description. The expression "mixture" also means a mixture of a cis isomer and trans isomer.

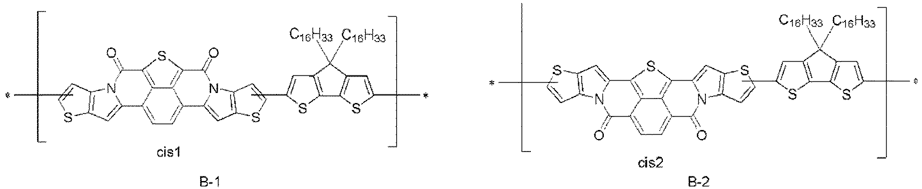

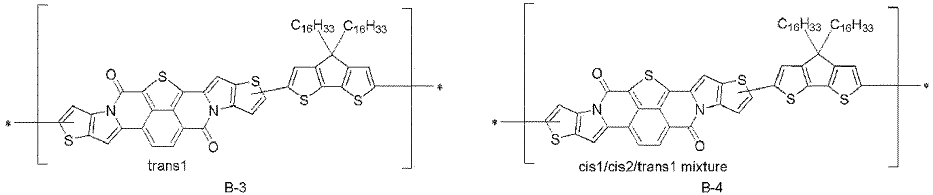

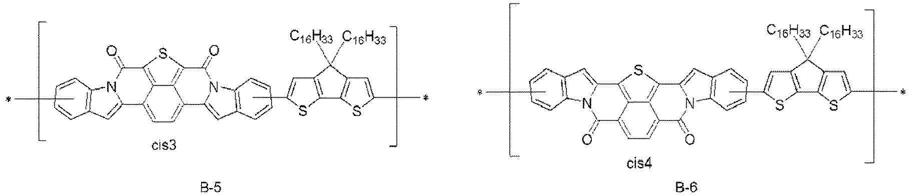

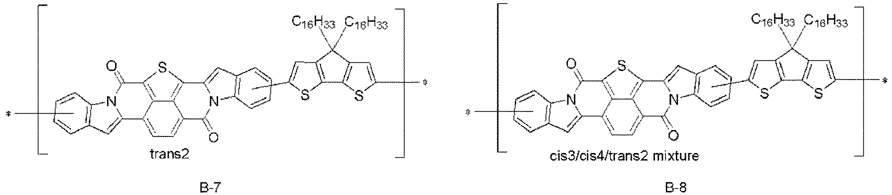

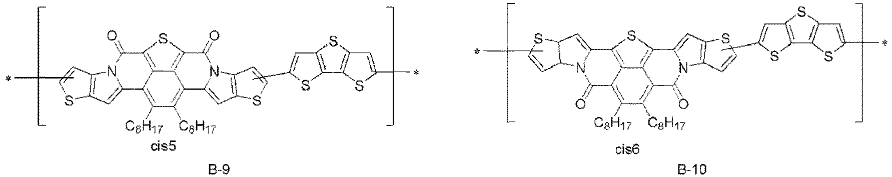

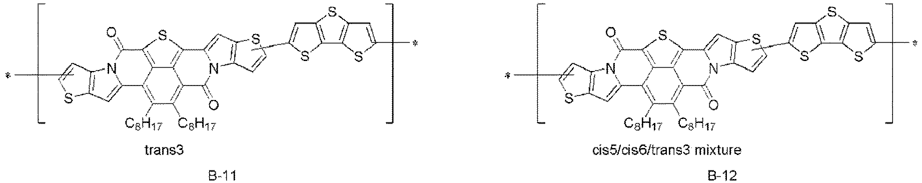

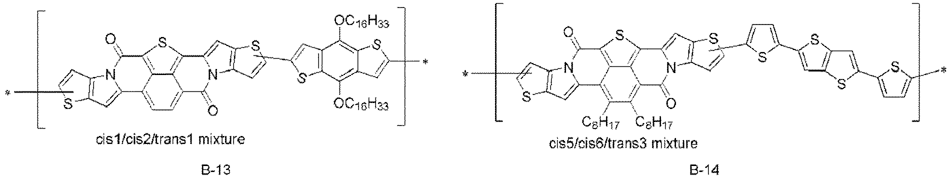

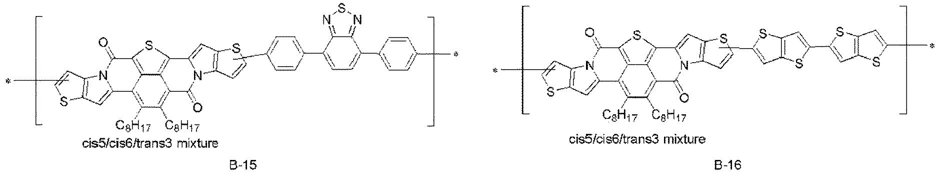

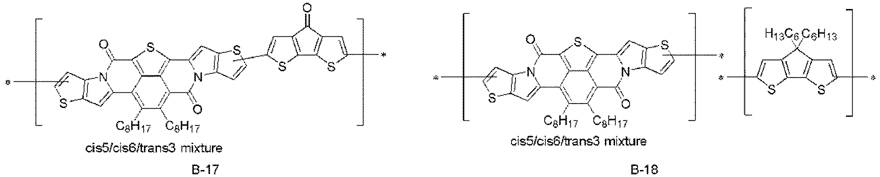

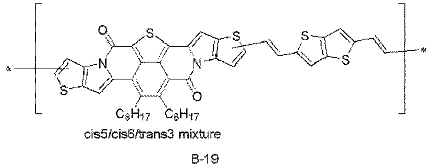

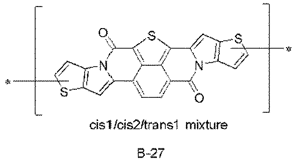

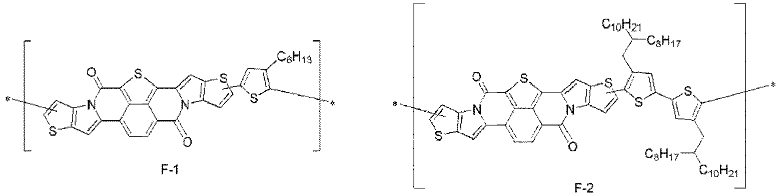

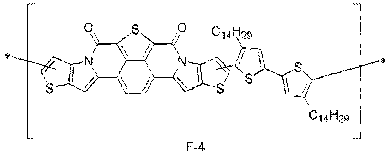

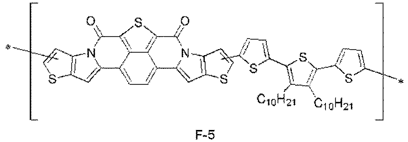

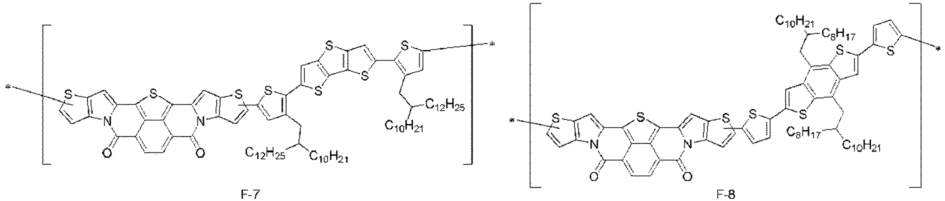

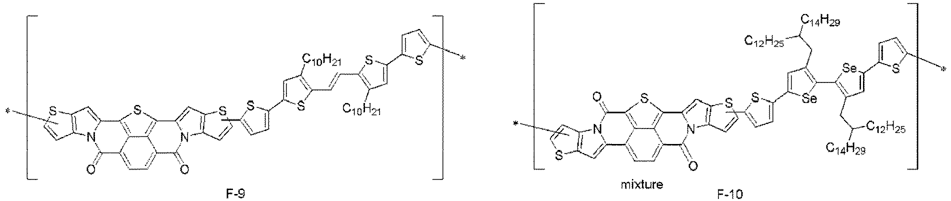

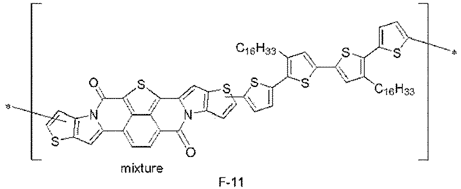

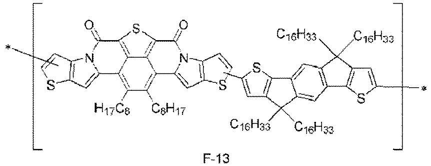

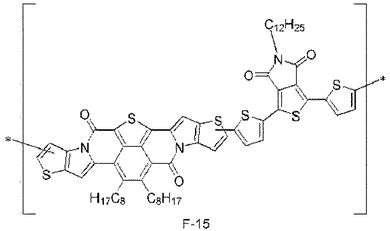

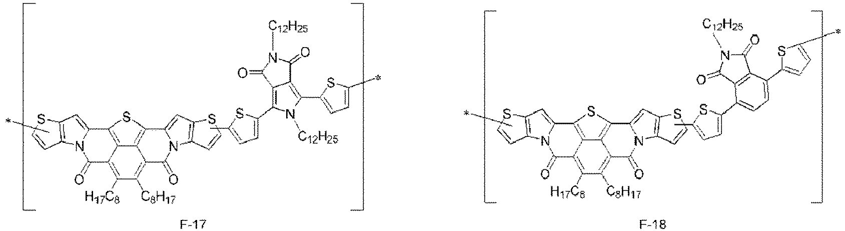

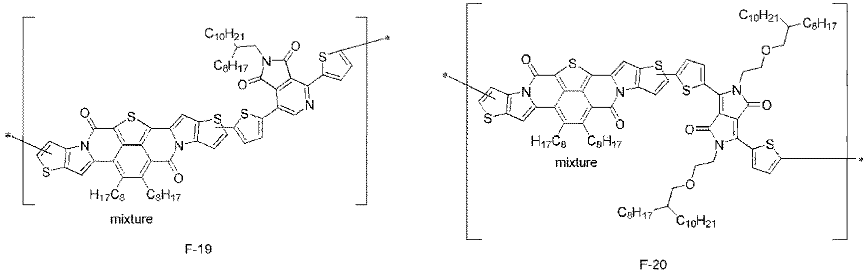

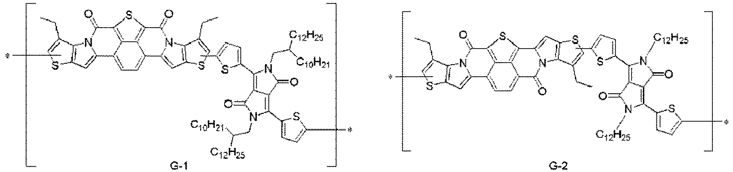

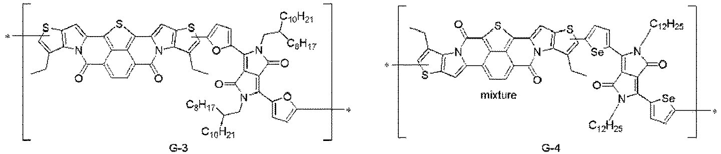

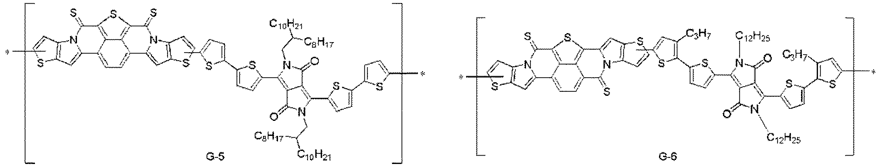

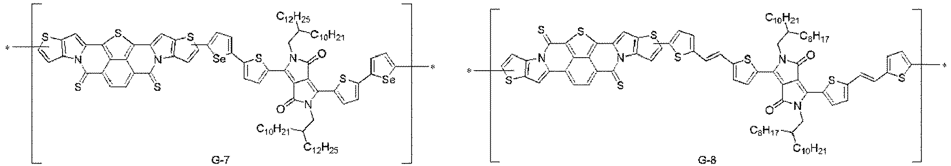

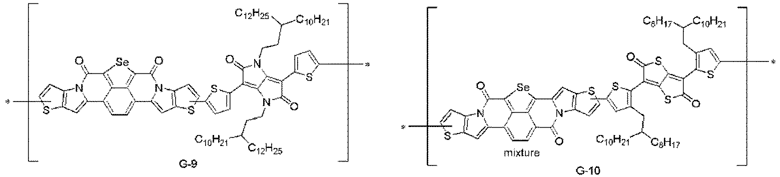

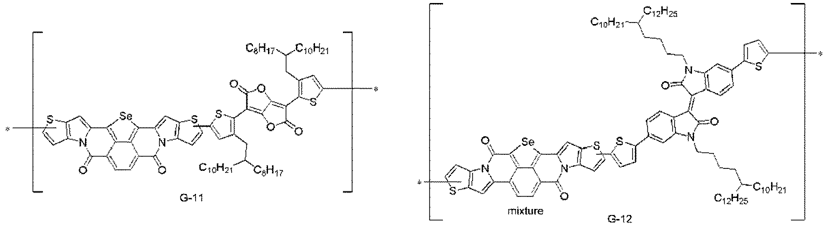

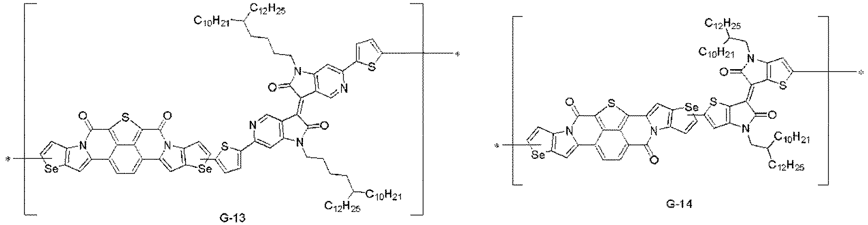

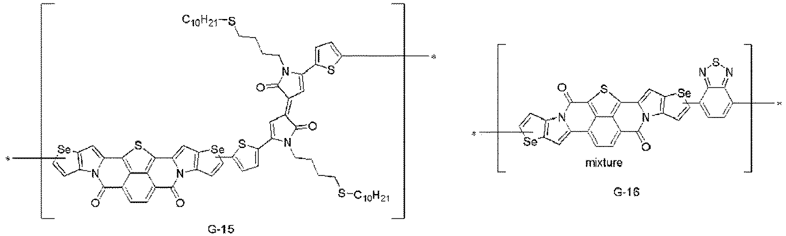

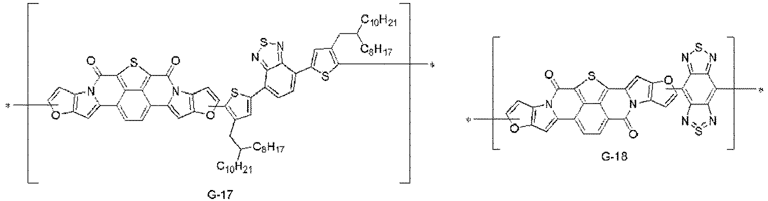

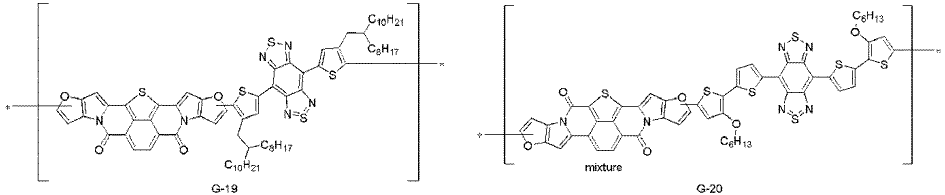

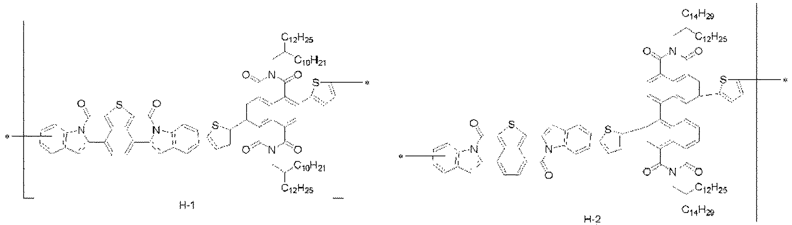

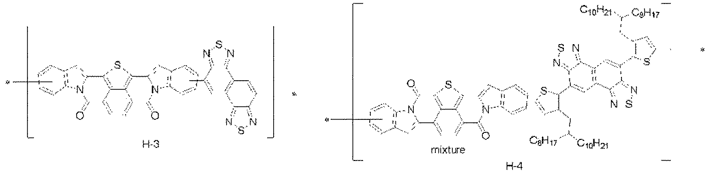

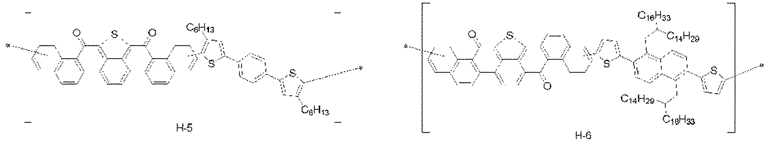

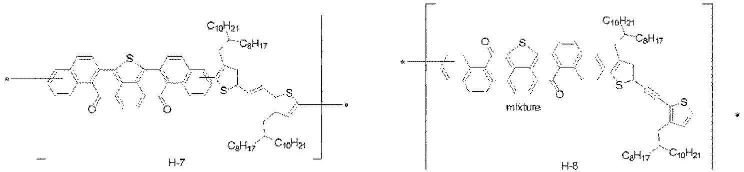

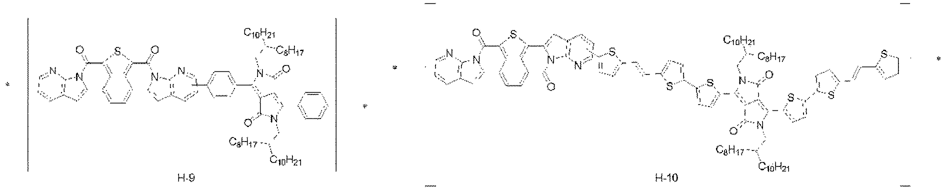

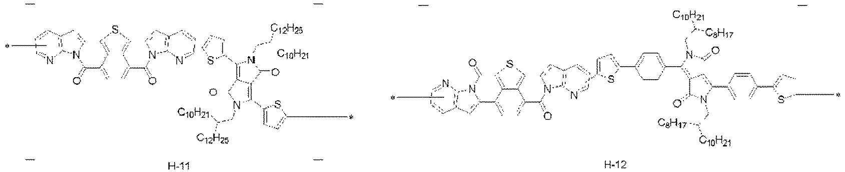

In a case where the organic semiconductor layer contains at least one compound of Formulae (1) and (2), the content of these compounds in the organic semiconductor layer is preferably ≥ 10 mass%, more preferably ≥ 30 mass%, and even more preferably ≥ 50 mass% in total. The total of the contents of the compounds of Formulae (1) and (2) in the organic semiconductor layer can be 100 mass%. In a case where the organic semiconductor film contains e.g a binder described below, for example, the total content is preferably ≤ 90 mass% and more preferably ≤ 80 mass%.

In a case where the organic semiconductor layer contains at least one structural unit of any of Formulae (9) and (10), the content of the polymer having at least one structural unit of any of Formulae (9) and (10) in the organic semiconductor layer is preferably ≥ 10 mass%, more preferably ≥ 30 mass%, and even more preferably ≥ 50 mass% in total. The total of the contents of this polymer in the organic semiconductor layer can be 100 mass%. In a case where the organic semiconductor film contains e.g. a binder described below, for example, the total content is preferably ≤ 90 mass% and more preferably ≤ 80 mass%.

A compound of any of Formulae (1) and (2) and a polymer having at least one structural units of any of Formulae (9) and (10) are collectively referred to as the "present organic semiconductor".

In addition to the present organic semiconductor, the organic semiconductor layer may contain a binder or an additive. As the additive, an additive that is generally used in the organic semiconductor layer may be used without particular limitation. The binder is described below.

### (Binder)

As the binder, a binder that is generally used in the organic semiconductor layer may be used without particular limitation.

Examples of the binder include an insulating polymer such as polystyrene, poly(α-methylstyrene), polyvinylcinnamate, poly(4-divinylbenzene), poly(4-vinylphenol), poly(4-methylstyrene), polycarbonate, polyarylate, polyester, polyamide, polyimide, polyurethane, polysiloxane, polysulfone, polymethyl methacrylate, polymethyl acrylate, cellulose, polyethylene, or polypropylene, and copolymers thereof, a semiconductive polymer such as polysilane, polycarbazole, polyarylamine, polyfluorene, polythiophene, polypyrrole, polyaniline, polyparaphenylene vinylene, polyacene, or polyheteroacene, and copolymers thereof, rubber, and a thermoplastic elastomer.

Among these, a polymer compound having a benzene ring (polymer having a repeating unit having a benzene ring group) is preferable. The content of the repeating unit having a benzene ring group is not particularly limited, but is preferably ≥ 50 mol%, more preferably ≥ 70 mol%, and even more preferably ≥ 90 mol% with respect to the total repeating units. The upper limit is not particularly limited, but examples of the upper limit include 100 mol%.

The weight-average molecular weight of the binder is used as polymer is not particularly limited, and preferably 1,000-10,000,000, more preferably 3,000-5,000,000, and even more preferably 5,000-3,000,000.

The binder and the additive may be contained singly, and two or more kinds thereof may be contained, respectively.

The content of the binder in the organic semiconductor layer can be appropriately set without particular limitation. For example, the content is preferably ≤ 90 mass%, more preferably ≤ 70 mass%, and even more preferably ≤ 50 mass%. The content of the binder in the organic semiconductor layer can be ≥ 0 mass%, and for example, is preferably ≥ 10 mass%, more preferably ≥ 15 mass%, and even more preferably ≥ 20 mass%.

The content of the additive in the organic semiconductor layer is preferably ≤ 10 mass%, more preferably ≤ 5 mass%, and even more preferably ≤ 1 mass%.

The film thickness of the organic semiconductor layer can be appropriately adjusted depending on the applied organic semiconductor element, and for example, is preferably 10-500 nm and more preferably 20-200 nm.

### (Method of forming organic semiconductor layer)

For example, the organic semiconductor layer can be formed by preparing a composition (hereinafter, also referred to as the "organic semiconductor composition of the present invention") obtained by dissolving the present organic semiconductor in a solvent, coating the substrate with the composition, and depositing the present organic semiconductor. More preferably, the substrate is coated with the present organic semiconductor composition to form the coating film, and the coating film is dried to form the organic semiconductor layer. The organic semiconductor composition can contain a binder and/or an additive. The content of the binder and the additive in the organic semiconductor composition may be appropriately adjusted depending on the aspect of the organic semiconductor layer to be formed.

In the present invention, the expression "coating the substrate with the organic semiconductor composition" includes an aspect of applying the organic semiconductor composition over the substrate through another layer provided on the substrate, in addition to an aspect of directly the organic semiconductor composition to the substrate. Another layer (a layer that is in contact with an organic semiconductor layer and becomes a base of the organic semiconductor layer) to be coated with the organic semiconductor composition is inevitably determined according to the structure of the organic thin film transistor element. For example, in the case of a bottom gate type, the layer is a gate insulating film, and in the case of a top gate type (top gate-bottom contact type and top gate-top contact type), the layer is a source electrode or a drain electrode.

Well-known methods can be used as the coating method with the organic semiconductor composition, and examples thereof include a bar coating method, a spin coating method, a knife coating method, a doctor blade method, an ink jet printing method, a flexographic printing method, a gravure printing method, or a screen printing method. As the coating method with the organic semiconductor composition, a method (so-called gap cast method) of forming an organic semiconductor film disclosed in JP-A-2013-207085 and a method (a so-called edge casting method and a continuous edge casting method) of manufacturing an organic semiconductor thin film disclosed in WO 2014/175351 can be suitably applied.

In the drying (drying treatment), conditions can be appropriately selected according to the kind of each component included in the organic semiconductor composition. Although natural drying may be used, in view of improving productivity, a heat treatment is preferable. The heat treatment conditions cannot be definitively determined, but for example, the heating temperature is preferably 30-250°C, more preferably 40-200°C, and even more preferably 50-150°C, and the heating time is preferably 10-300 minutes and more preferably 20-180 minutes.

The method of preparing the present organic semiconductor composition is not particularly limited, and a general preparation method may be employed. For example, it is possible to prepare the present organic semiconductor composition by adding respective components in a predetermined amount to the solvent and appropriately performing a stirring treatment.

The solvent is not particularly limited as long as the solvent dissolves or disperses the above polymer, and examples thereof include an inorganic solvent and an organic solvent. Among these, an organic solvent is preferable. The solvent may be used singly, and two or more kinds thereof may be used in combination.

The organic solvent is not particularly limited, and examples thereof include a hydrocarbon solvent such as hexane, octane, and decane, an aromatic hydrocarbon solvent such as toluene, xylene, mesitylene, ethylbenzene, decalin, 1-methylnaphthalene, tetralin, and anisole, a ketone solvent such as acetone, methyl ethyl ketone, methyl isobutyl ketone, and cyclohexanone, a halogenated hydrocarbon solvent such as dichloromethane, chloroform, tetrachloromethane, dichloroethane, trichloroethane, tetrachloroethane, chlorobenzene, dichlorobenzene, and chlorotoluene, an ester solvent such as ethyl acetate, butyl acetate, amyl acetate, and ethyl lactate, an alcohol solvent such as methanol, propanol, butanol, pentanol, hexanol, cyclohexanol, methyl cellosolve, ethyl cellosolve, and ethylene glycol, an ether solvent such as butoxybenzene, dibutyl ether, tetrahydrofuran, and dioxane, an amide solvent such as N,N-dimethylformamide and N,N-dimethylacetamide, an imide solvent such as 1-methyl-2-pyrrolidone and 1-methyl-2-imidazolidinone, a sulfoxide solvent such as dimethylsulfoxide, and a nitrile solvent such as acetonitrile.

The content ratio of the solvent in the organic semiconductor composition is preferably 90-99.99 mass%, more preferably 95-99.99 mass%, and even more preferably 96-99.95 mass%.

### (Sealing layer)

As described above, the organic semiconductor layer contains the present organic semiconductor, and exhibits high heat resistance. Accordingly, even in a case where a heating step such as formation of e.g. a sealing layer is performed after the organic semiconductor is provided, carrier mobility can be maintained at a desired level.

Accordingly, in view of durability, the present organic thin transistor element preferably includes a sealing layer as an outermost layer. As a result, both of the excellent carrier mobility and the excellent durability can be sufficiently achieved.

For the sealing layer, a sealing agent (composition for forming a sealing layer) generally used for an organic TFT element can be used.

The sealing agent is preferably heated and dried to form a layer. The heating conditions at this point cannot be definitively determined, depending on the kind of e.g. the sealing agent, but for example, the heating temperature is preferably 50-200°C, and more preferably 100-175°C. Other conditions such as the heating time are appropriately determined according to the kind of e.g. the sealing agent.

The thickness of the sealing layer is not particularly limited but is preferably 0.2-10 µm.

### -Bottom gate-top contact-type organic thin film transistor element-

Fig. 2 is a cross-sectional schematic view indicating a bottom gate-top contact-type organic thin film transistor element 200 which is an example of the present semiconductor element.

As illustrated in Fig. 2, the organic thin film transistor element 200 has a base material 10, a gate electrode 20, a gate insulating film 30, a source electrode 40, a drain electrode 42, an organic semiconductor film 50, and a sealing layer 60.

The organic thin film transistor element 200 is the same as the organic thin film transistor element 100 except that the layer configuration (lamination aspect) is different. Accordingly, the substrate, the gate electrode, the gate insulating film, the source electrode, the drain electrode, the organic semiconductor layer, and the sealing layer are the same as those of the bottom gate-bottom contact-type organic thin film transistor element, and thus descriptions thereof are omitted.

### Examples

The present invention is more specifically described based on the examples. In this example, Et is ethyl, Me is methyl, Ph is phenyl, Bu is butyl, Ac is acetyl, and TBDPS is tert-butyldiphenylsilyl. DMF is N,N-dimethylformamide, THF is tetrahydrofuran, DMSO is dimethyl sulfoxide, NBS is N-bromosuccinimide, TFA is trifluoroacetic acid, NMP is N-methyl-2-pyrrolidone, DME is dimethyl ether, and TMEDA is tetramethyl ethylene diamine.

### [Synthesis of Intermediate 1]

J. Mater. Chem., 2012, Book 22, p. 23514 and Org. Lett. 2012, Book 14, p. 3100, were referred to, and according to Scheme 1, an intermediate 1 (3.4 g, 12.4 mmol) was obtained from a compound 1-1 (20 g, 115 mmol).

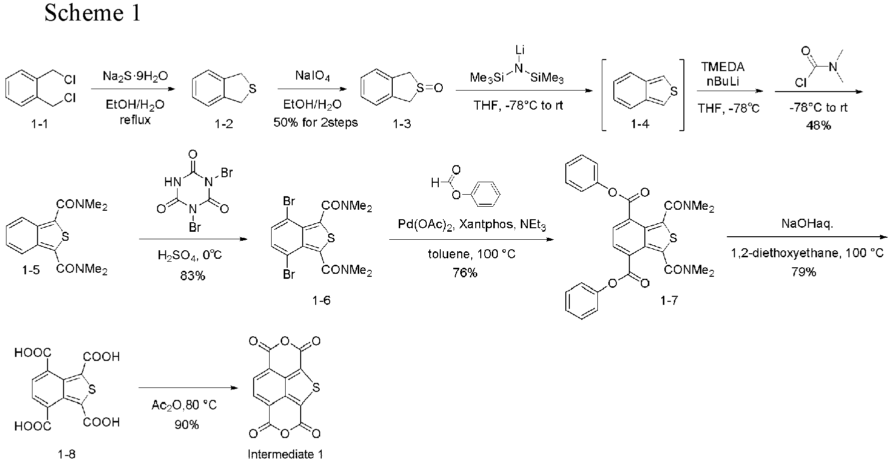

### [Synthesis of Intermediate 2]

Tetrahedron Letters, 2013, Book 54, p. 3171 was referred to, to obtain an intermediate 2 (573 mg, 1.15 mmol), from a compound 1-7 (5.0 g, 9.68 mmol) according to Scheme 2.

In Scheme 2, Ph represents phenyl, and rt is 25°C.

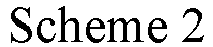

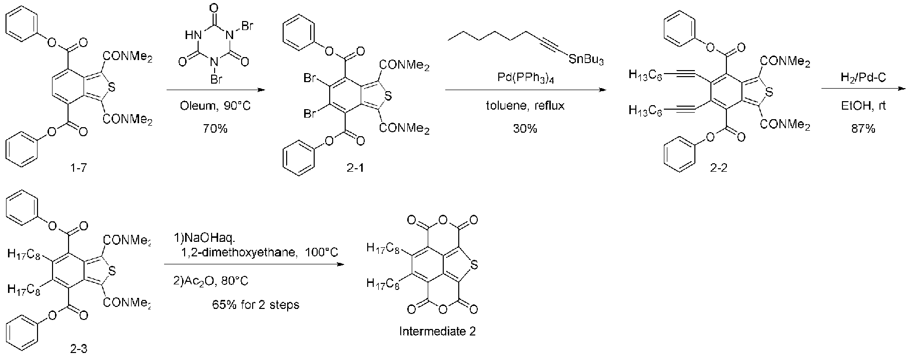

### [Synthesis of Intermediate 3] (Reference, not according to the invention)

An intermediate 3 was obtained in the same manner as in Scheme 1, except that a compound 3-1 was used instead of the compound 1-1.

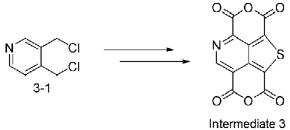

### [Synthesis of compounds 4-5 to 4-7] (Reference, not according to the invention)

New J. Chem. 2010, Book 34, p. 236was referred to, and according to Scheme 3, compounds 4-5 (159 mg, 0.371 mmol), 4-6 (191 mg, 0.445 mmol), and 4-7 (143 mg, 0.334 mmol) were synthesized from intermediate 3 (2 g, 7.27 mmol). The compounds 4-5, 4-6, and 4-7 were separated by silica gel column chromatography.

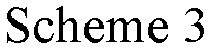

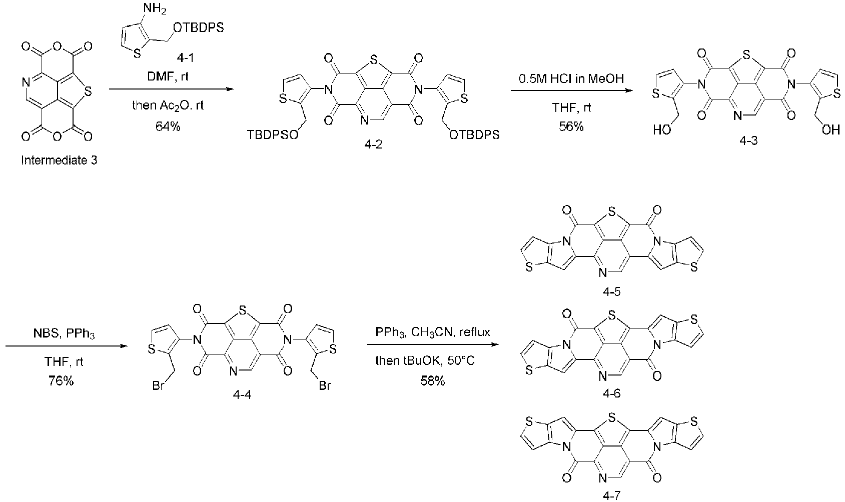

### [Synthesis of compound A-1] (Reference, not according to the invention)

New J. Chem. 2010, Book 34, p. 236was referred to, and according to Scheme 4, a compound A-1 was synthesized

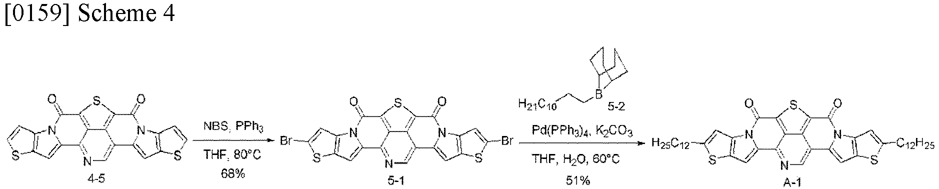

### [Synthesis of compounds A-4 to A-7]

Compounds A-4 to A-7 were obtained in the same manner as in Schemes 3 and 4, except that intermediate 1 (compounds A-4 to A-6) or intermediate 2 (compound A-7) was used instead of intermediate 3. The isomers were separated by silica gel column chromatography.

### [Synthesis of compound A-8]

Bull Acad Sci USSR, 1981, Book 30, p. 619was referred to, and a compound 6-3 was synthesized according to Scheme 5.

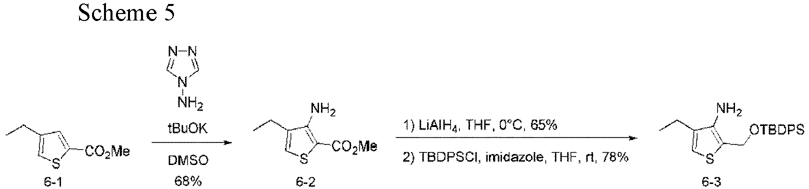

A compound A-8 was obtained in the same manner as in Schemes 3 and 4, except that a compound 6-3 was used instead of the compound 4-1, and an intermediate 1 was used instead of the intermediate 3. The isomers were separated by silica gel column chromatography.

### [Synthesis of compound A-9]

Tetrahedron Letters, 2013, Book 54, p. 7103 was referred to, and according to Scheme 6, a compound A-9 (65.8 mg, 0.0825 mmol) was obtained from a compound A-4 (100 mg, 0.131 mmol).

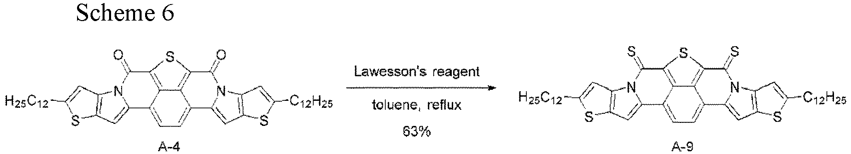

### [Synthesis of compounds A-12 to A-14]

Compounds A-12 to A-14 were obtained in the same manner as in the above scheme, except that compounds 7-1, 7-2, and 7-3 disclosed in Tetrahedron Letters, 1990, Book 31, p. 3155, Org. Lett. 2003, Book 5, p. 2519, or J. Am. Chem. Soc. 2009, Book 131, p. 6070 were respectively used instead of the compound 1-4. The isomers were separated by silica gel column chromatography.

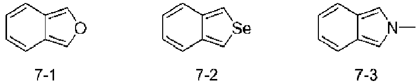

### [Synthesis of compound A-15]

Chem. Lett. 2014, Book 43, p. 293 and Synthesis 2015, Book 47, p. 3049 were referred to, and according to Scheme 9, a compound A-15 (629 mg, 0.811 mmol) was obtained from the compound 1-6 (1 g, 2.30 mmol).

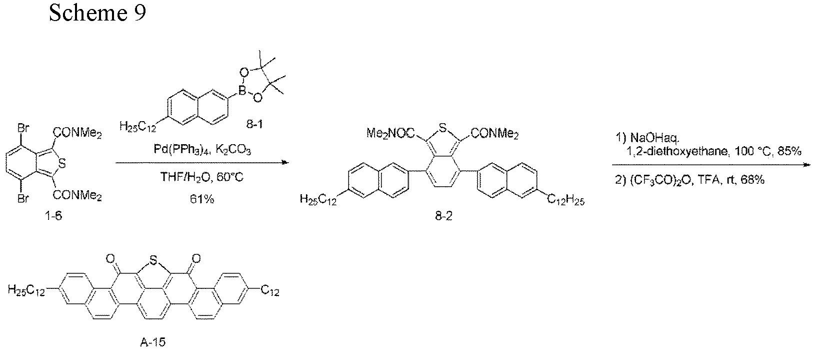

### [Synthesis of compound A-16]

A compound A-16 was obtained in the same manner as in Scheme 9, except that a compound 9-1 was used instead of the compound 8-1.

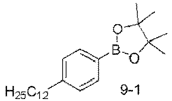

### [Synthesis of compound A-17]

J. Am. Chem. Soc. 2003, Book 125, p. 5274 was referred to, and according to Scheme 10, a compound A-17 (828 mg, 1.27 mmol) was obtained from a compound 1-6 (1 g, 2.30 mmol).

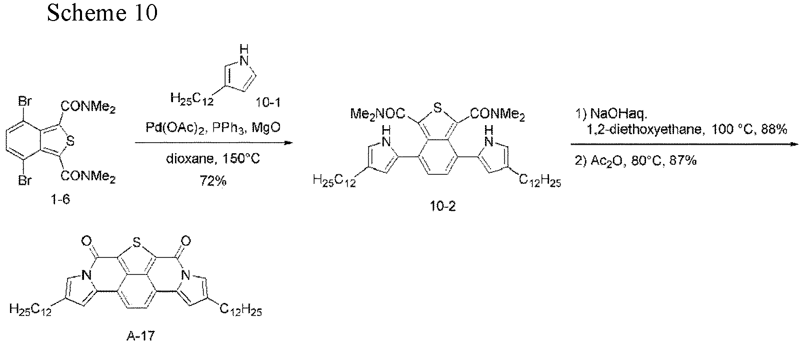

### [Synthesis of compound A-18]

A compound A-18 was obtained in the same manner as in Schemes 3 and 4, except that the compound 11-1 synthesized with reference to Angew. Chem. Int. Ed. 2014, Book 53, p. 9603 was used instead of the compound 4-1, and the intermediate 1 was used instead of the intermediate 3. The isomers were separated by silica gel column chromatography.

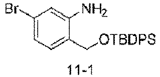

### [Synthesis of compounds A-19 and A-20]

Compounds A-19 and A-20 were obtained in the same manner as in Schemes 3 and 4, except that the intermediate 1 was used instead of the intermediate 3, and compounds 12-1 and 12-2 were respectively used instead of the compound 4-1. The isomers were separated by silica gel column chromatography.

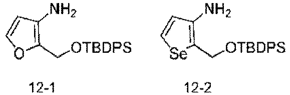

### [Synthesis of compounds A-21 to A-29]

Compounds A-21 to A-29 were obtained in the same manner as in Schemes 3 and 4, except that the intermediate 1 was used instead of the intermediate 3, and corresponding compounds were used instead of the compound 5-2. The isomers were separated by silica gel column chromatography.

### [Synthesis of compound A-30]

A compound A-30 was obtained in the same manner as in Scheme 10, except that the compound 13-1 synthesized with reference to Bull. Chem. Soc. Jpn. 1992, Book 65, p. 2992 was used instead of the compound 10-1.

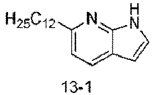

### [Comparative Synthesis Example 1]

Comparative compounds 1 and 2 were synthesized according to the following scheme.

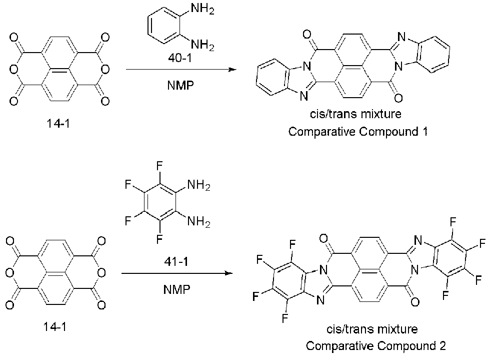

The structures of the obtained compounds A-4 to A-9 and A12 to A-30 are shown in the table provided earlier herein. The structures of the obtained comparative compounds 1 and 2 are as follows:

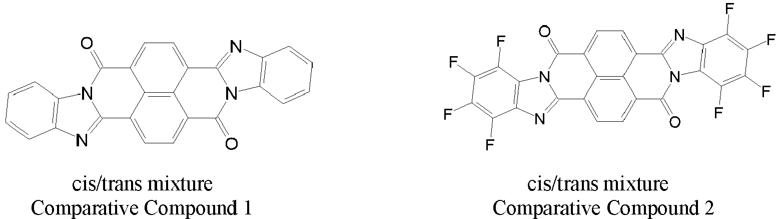

Each of the above organic semiconductor compounds and comparative compounds was examined for purity (area ratio of absorption strength at 254 nm) by high performance liquid chromatography (TSKgel ODS-100Z manufactured by Tosoh Corporation), and the purity was ≥ 99.0% or more.

### [Manufacturing of bottom gate-top contact element by vapor deposition process]

A surface of a 10 mm × 10 mm substrate on the thermal oxide film side in which a SiO 2 thermal oxide film of 500 nm was formed on the surface of an n-type silicon substrate (having a thickness of 0.4 mm) was subjected to ultraviolet light (UV)-ozone cleaning and was treated with β-phenytillymethoxysilane.

Films were formed on the β-phenylitylmethoxysilane-treated surface of this substrate by vapor deposition with the compounds presented in the following table at a deposition rate of 0.05 nm/s so as to have a film thickness of 40 nm.

Organic thin film transistor elements 1-4 to 1-34 (hereinafter, also referred to as "elements 1-4 to 1-34") and comparative organic thin film transistor elements 1-1 and 1-2 (hereinafter, also referred to as "comparative elements 1-1 and 1-2") for FET characteristic measurement were obtained by attaching a mask on the obtained organic semiconductor films and performing vapor deposition of 2 nm of F4-TCNQ and 40 nm of a gold electrode as charge injection acceptors.

### [Evaluation of carrier mobility]

With respect to the FET characteristic of each organic thin film transistor element (elements 1-4 to 1-34 and comparative elements 1-1 and 1-2), the carrier mobility under atmospheric pressure was evaluated by using a semiconductor parameter analyzer (4156C manufactured by Agilent Technologies, Inc.) connected to a semi-auto prober (AX-2000 manufactured by Vector Semiconductor Co., Ltd.).

The carrier mobility µ was obtained by applying a voltage of -50 V between the source electrode and the drain electrode of each organic thin film transistor element (FET element), changing the gate voltage in the range of 20 V to -150 V, and using an expression I_{d=}(W/2L)µCᵢ(V_{g}-Vₜₕ)² (in the expression, L represents a gate length, W represents a gate width, Ci represents a capacitance per unit area of an insulating layer, V_{g} represents a gate voltage, and Vₜₕ represents a threshold voltage) representing drain current I_{d}, and was evaluated by the following evaluation standards.

### -Evaluation of carrier mobility-

AA: ≥ 1.0 cm²/Vs
A: 0.8 to < 1.0 cm²/Vs
B: 0.6 to < 0.8 cm²/Vs
C: 0.4 to < 0.6 cm²/Vs
D: 0.2 to < 0.4 cm²/Vs
E: 0.1 to < 0.2 cm²/Vs
F: 0.05 to < 0.1 cm²/Vs
G: < 0.05 cm²/Vs

The results thereof are presented in the following table.

### [Evaluation of heat resistance]

Each of the elements 1-4 to 1-34 and the comparative elements 1-1 and 1-2 was heated at 150°C for 30 minutes in the atmosphere, and then the carrier mobility was evaluated in the same manner as described above. A maintenance rate (%) of the carrier mobility was calculated by applying the carrier mobility before and after heating to the following equation.

Maintenance rate (%) of carrier mobility = 100 × {carrier mobility (after heating) / carrier mobility (before heating)}

The maintenance rate of the carrier mobility was applied to the following evaluation standards to evaluate the heat resistance.

### -Evaluation standard of maintenance rate of carrier mobility (heat resistance)-

AA: ≥ 90%
A: 80 to < 90%
B: 70 to < 80%
C: 60 to < 70%
D: 50 to < 60%
E: 40 to < 50%
F: 30 to < 40%
G: < 30%

The results thereof are presented in the following table.

**[Table 1]**

| | Element No. | Compound in organic semiconductor layer | Carrier mobility | Heat resistance |
|---|---|---|---|---|
| Example 1-4 | Element 1-4 | A-4 | AA | AA |
| Example 1-5 | Element 1-5 | A-5 | AA | AA |
| Example 1-6 | Element 1-6 | A-6 | AA | AA |
| Example 1-7 | Element 1-7 | A-4 / A-5 = 1/ 1 (Molar ratio) | AA | AA |
| Example 1-8 | Element 1-8 | A-5 / A-6= 1 / 1 (Molar ratio) | AA | AA |
| Example 1-9 | Element 1-9 | A-6 / A-7= 1 / 1 (Molar ratio) | AA | AA |
| Example 1-10 | Element 1-10 | A-4 / A-5 / A-6 = 1 / 1 / 1 (Molar ratio) | AA | AA |
| Example 1-11 | Element 1-11 | A-7 | AA | AA |
| Example 1-12 | Element 1-12 | A-8 | AA | AA |
| Example 1-13 | Element 1-13 | A-9 | AA | AA |
| Example 1-16 | Element 1-16 | A-12 | AA | AA |
| Example 1-17 | Element 1-17 | A-13 | AA | AA |
| Example 1-18 | Element 1-18 | A-14 | AA | AA |
| Example 1-19 | Element 1-19 | A-15 | C | C |
| Example 1-20 | Element 1-20 | A-16 | C | D |
| Example 1-21 | Element 1-21 | A-17 | C | D |
| Example 1-22 | Element 1-22 | A-18 | B | B |
| Example 1-23 | Element 1-23 | A-19 | AA | AA |
| Example 1-24 | Element 1-24 | A-20 | AA | AA |
| Example 1-25 | Element 1-25 | A-21 | AA | AA |
| Example 1-26 | Element 1-26 | A-22 | AA | AA |
| Example 1-27 | Element 1-27 | A-23 | AA | AA |
| Example 1-28 | Element 1-28 | A-24 | AA | AA |
| Example 1-29 | Element 1-29 | A-25 | AA | AA |
| Example 1-30 | Element 1-30 | A-26 | AA | AA |
| Example 1-31 | Element 1-31 | A-27 | AA | AA |
| Example 1-32 | Element 1-32 | A-28 | A | A |
| Example 1-33 | Element 1-33 | A-29 | A | A |
| Example 1-34 | Element 1-34 | A-30 | C | C |
| Comparative Example 1-1 | Comparative Element 1-1 | Comparative Compound 1 | G | G |
| Comparative Example 1-2 | Comparative Element 1-2 | Comparative Compound 2 | G | G |

As presented in Table 1, even in a case where the organic semiconductor layer was an element including a compound having a fused polycyclic structure, in a case where the structure of the compound was out of the range determined in the present invention, the carrier mobility was inferior, and the heat resistance was inferior, as a result (Comparative Examples 1-1 and 1-2).

Meanwhile, it is understood that an element in which a compound having a structure determined in the present invention was used for the organic semiconductor layer had excellent carrier mobility and also excellent heat resistance (Examples 1-4 to 1-34).

### [Preparation of organic semiconductor composition]

Each compound presented in the following table, a composition obtained by mixing poly α-methylstyrene at a mass ratio of 1 : 1, and toluene as a solvent were mixed to prepare a solution (organic semiconductor composition) containing a compound at a concentration of 0.1 mass%. This solution was heated to 40°C and used to form the following element.

### [Manufacturing of bottom gate-bottom contact element using polymer binder]

The organic semiconductor composition prepared above was cast (drop cast method) to a substrate for a field effect transistor (FET) characteristic measurement which was heated to 40°C in a nitrogen atmosphere, so as to obtain organic thin film transistor elements 2-4 to 2-34 (hereinafter, also referred to as "elements 2-4 to 2-34") and comparative organic thin film transistor elements 2-1 and 2-2 (hereinafter, also referred to as "comparative elements 2-1 and 2-2").

As the substrate for FET characteristic measurement, a silicon substrate having a bottom gate and bottom contact structure comprising chromium/gold (gate width W = 100 mm, gate length L = 100 µm) disposed in a comb shape as source and drain electrodes and SiO₂ (film thickness of 500 nm) as an insulating film was used.

The carrier mobility and the heat resistance of each of the obtained elements were evaluated in the same manner as described above.

**[Table 2]**

| | Element No. | Compound in organic semiconductor layer | Carrier mobility | Heat resistance |
|---|---|---|---|---|
| Example 2-4 | Element 2-4 | A-4 | AA | AA |
| Example 2-5 | Element 2-5 | A-5 | AA | AA |
| Example 2-6 | Element 2-6 | A-6 | AA | AA |
| Example 2-7 | Element 2-7 | A-4 / A-5 = 1 / 1 (Molar ratio) | AA | AA |
| Example 2-8 | Element 2-8 | A-5 / A-6 = 1 / 1 (Molar ratio) | AA | AA |
| Example 2-9 | Element 2-9 | A-6 / A-7 = 1 / 1 (Molar ratio) | AA | AA |
| Example 2-10 | Element 2-10 | A-4 / A-5 / A-6 = 1 / 1 / 1 (Molar ratio) | AA | AA |
| Example 2-11 | Element 2-11 | A-7 | AA | AA |
| Example 2-12 | Element 2-12 | A-8 | AA | AA |
| Example 2-13 | Element 2-13 | A-9 | AA | AA |
| Example 2-16 | Element 2-16 | A-12 | AA | AA |
| Example 2-17 | Element 2-17 | A-13 | AA | AA |
| Example 2-18 | Element 2-18 | A-14 | AA | AA |
| Example 2-19 | Element 2-19 | A-15 | C | C |
| Example 2-20 | Element 2-20 | A-16 | C | D |
| Example 2-21 | Element 2-21 | A-17 | C | D |
| Example 2-22 | Element 2-22 | A-18 | B | B |
| Example 2-23 | Element 2-23 | A-19 | AA | AA |
| Example 2-24 | Element 2-24 | A-20 | AA | AA |
| Example 2-25 | Element 2-25 | A-21 | AA | AA |
| Example 2-26 | Element 2-26 | A-22 | AA | AA |
| Example 2-27 | Element 2-27 | A-23 | AA | AA |
| Example 2-28 | Element 2-28 | A-24 | AA | AA |
| Example 2-29 | Element 2-29 | A-25 | AA | AA |
| Example 2-30 | Element 2-30 | A-26 | AA | AA |
| Example 2-31 | Element 2-31 | A-27 | AA | AA |
| Example 2-32 | Element 2-32 | A-28 | A | A |
| Example 2-33 | Element 2-33 | A-29 | A | A |
| Example 2-34 | Element 2-34 | A-30 | C | C |
| Comparative Example 2-1 | Comparative Element 2-1 | Comparative Compound 1 | G | G |
| Comparative Example 2-2 | Comparative Element 2-2 | Comparative Compound 2 | G | G |

As presented in Table 2, even in a case where the organic semiconductor layer was an element including a compound having a fused polycyclic structure, in a case where the structure of the compound was out of the range determined in the present invention, the carrier mobility was inferior, and the heat resistance was inferior, as a result (Comparative Examples 2-1 and 2-2).

Meanwhile, it is understood that an element in which a compound having a structure determined in the present invention was used for the organic semiconductor layer had excellent carrier mobility and also excellent heat resistance (Examples 2-4 to 2-34).

### [Synthesis of compound 16-1]

A compound 16-2 (848 mg, 1.45 mmol) was obtained from a compound 16-1 (2 g, 4.67 mmol) which was an intermediate in the synthesis of compound A-4 according to Scheme 11.

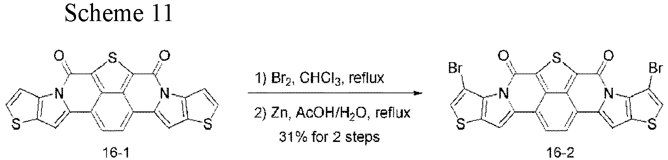

### [Synthesis of compounds 17-1 to 17-7]

Compounds 17-1 and 17-2 were obtained from intermediates in the synthesis of the compound A-4, in the same manner as in Scheme 11.

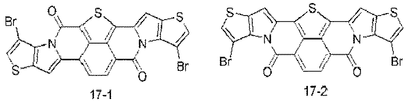

### [Synthesis of compounds 18-2 to 18-4]

Compounds 18-2 to 18-4 were obtained in the same manner as in Schemes 3 and 4, except that the compound 18-1 synthesized with reference to Tetrahedron Letters 2002, Book 43, p. 1171 was used instead of the compound 4-1. The isomers were separated by silica gel column chromatography.

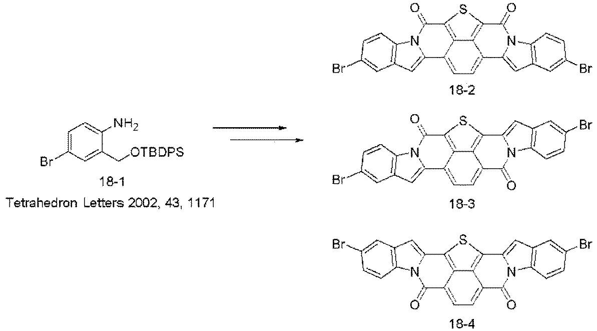

### [Synthesis of compounds 19-1 to 19-3]

Compounds 19-1 to 19-3 were obtained from the intermediate in the synthesis of the compound A-7 in the same manner as in Scheme 11.

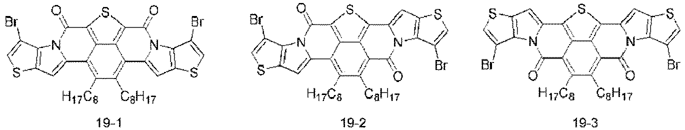

### [Synthesis of polymer B-1]

With reference to US9293708B, as described below, a polymer B-1 (1.5 g) was synthesized from a compound 16-2/20-1 (1 g in total) and a compound 20-2 according to Scheme 12 at the reaction temperature of 80°C and the reaction time of two minutes. The compound 20-1 was an intermediate in the synthesis of the compound A-4. The weight-average molecular weight (Mw) of the polymer B-1 (a) was 3,100.

The preparation amounts of the compounds 16-2 and 20-1 were equal in mass ratio. Similarly, in the subsequent polymer synthesis, in a case where a plurality of compounds were used as a compound for deriving the structural unit represented by any one of Formulae (9) and (10), the preparation amounts thereof were all equal in mass ratio.

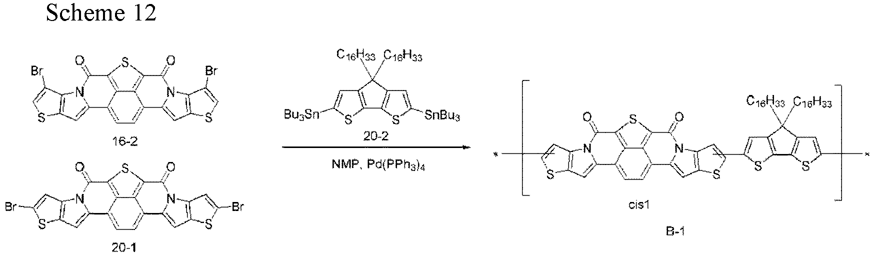

A polymer B-1 was obtained in the same manner except that the reaction temperature was 80°C and the reaction time was 30 minutes. Mw of the polymer B-1 (b) was 11,900.

The polymer B-1 was obtained in the same manner as in Scheme 12, except that the reaction temperature was 100°C and the reaction time was three hours. Mw of the polymer B-1 (C) was 48,500.

The polymer B-1 was obtained in the same manner as in Scheme 12, except that the reaction temperature was 120°C and the reaction time was three hours. Mw of the polymer B-1 (d) was 97,100.

The polymer B-1 was obtained in the same manner as in Scheme 12, except that the reaction temperature was 120°C and the reaction time was six hours. Mw of the polymer B-1 (e) was 143,200.

### [Synthesis of polymer B-2]

A polymer B-2 was obtained in the same manner as in Scheme 12, except that compounds 17-2 and 21-1 were used instead of the compounds 16-2 and 20-1. The compound 21-1 was an intermediate in the synthesis of the compound A-4.

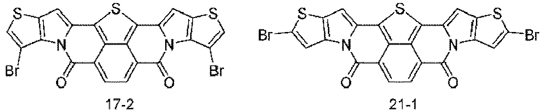

### [Synthesis of polymer B-3]

A polymer B-3 was obtained in the same manner as in Scheme 12, except that compounds 17-1 and 22-1 were used instead of the compounds 16-2 and 20-1. The compound 22-1 was an intermediate in the synthesis of the compound A-4.

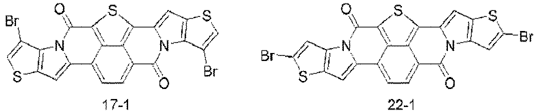

### [Synthesis of polymer B-4]

A polymer B-4 was obtained in Scheme 12 by using compounds 16-2, 20-1, 17-2, 21-1, 17-1, and 22-1 instead of the compounds 16-2 and 20-1.

### [Synthesis of polymer B-5]

A polymer B-5 was obtained in the same manner as in Scheme 12, except that compounds 18-2 and 23-1 were used instead of the compounds 16-2 and 20-1. The compound 23-1 was an intermediate in the synthesis of the compound A-18.

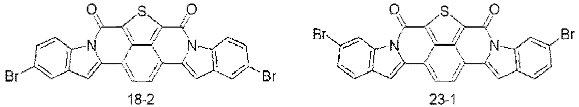

### [Synthesis of polymer B-6]

A polymer B-6 was obtained in the same manner as in Scheme 12, except that compounds 18-4 and 24-1 were used instead of the compounds 16-2 and 20-1. The compound 24-1 was an intermediate in the synthesis of the compound A-18.

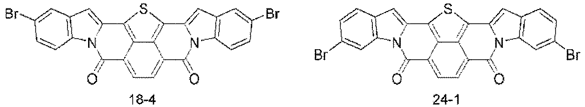

### [Synthesis of polymer B-7]

A polymer B-7 was obtained in the same manner as in Scheme 12, except that compounds 18-3 and 25-1 were used instead of the compounds 16-2 and 20-1. The compound 25-1 was an intermediate in the synthesis of the compound A-18.

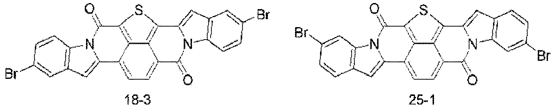

### [Synthesis of polymer B-8]

A polymer B-8 was obtained in Scheme 12 by using compounds 18-2, 23-1, 18-4, 24-1, 18-3, and 25-1 instead of the compounds 16-2 and 20-1.

### [Synthesis of polymer B-9]

A polymer B-9 was obtained in the same manner as in Scheme 12, except that the compounds 19-1 and 26-1 were used instead of the compounds 16-2 and 20-1, and a compound 26-2 synthesized with reference to J. Polym. Sci., A Polym. Chem. 2013, Book 51, p. 1933 was used instead of the compound 20-2. The compound 26-1 was an intermediate in the synthesis of the compound A-7.

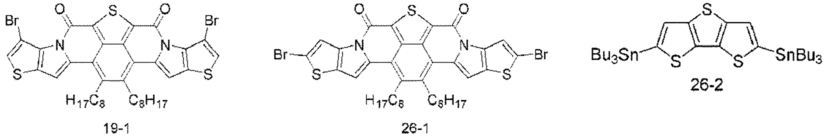

### [Synthesis of polymer B-10]

A polymer B-10 was obtained in the same manner as in Scheme 12, except that compounds 19-3 and 27-1 were used instead of the compounds 16-2 and 20-1, and a compound 26-2 was used instead of the compound 20-2. The compound 27-1 was an intermediate in the synthesis of the compound A-7.

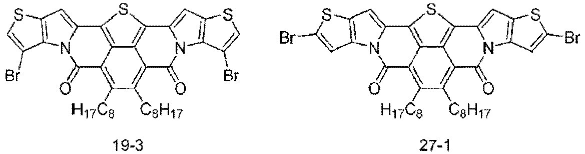

### [Synthesis of polymer B-11]

A polymer B-11 was obtained in the same manner as in Scheme 12, except that the compounds 19-2 and 28-1 were used instead of the compounds 16-2 and 20-1, and a compound 26-2 was used instead of the compound 20-2. The compound 28-1 was an intermediate in the synthesis of the compound A-7.

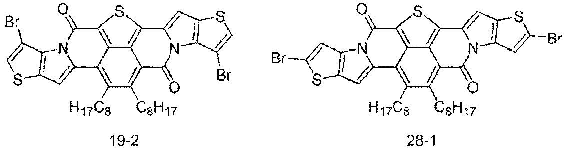

### [Synthesis of polymer B-12]

In Scheme 12, a polymer B-12 was obtained in the same manner as in Scheme 12, except that the compounds 19-1, 26-1, 19-3, 27-1, 19-2, and 28-1 were used instead of the compounds 16-2 and 20-1, and a compound 26-2 was used instead of the compound 20-2.

### [Synthesis of polymer B-13]

In Scheme 12, a polymer B-13 was obtained in the same manner as in Scheme 12, except that the compounds 16-2, 20-1, 17-2, 21-1, 17-1, and 22-1 were used instead of the compounds 16-2 and 20-1, and a compound 29-1 synthesized with reference to J. Am. Chem. Soc. 2013, Book 135, p. 4656 was used instead of the compound 20-2.

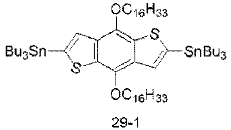

### [Synthesis of polymer B-14]

In Scheme 12, a polymer B-14 was obtained in the same manner as in Scheme 12, except that the compounds 19-1, 26-1, 19-3, 27-1, 19-2, and 28-1 were used instead of the compounds 16-2 and 20-1, and a compound 30-1 synthesized with reference to Molecules 2012, Book 17, p. 12163 was used instead of the compound 20-2.

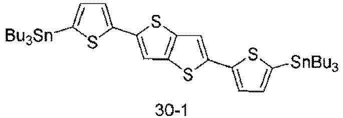

### [Synthesis of polymer B-15]

In Scheme 12, a polymer B-15 was obtained in the same manner as in Scheme 12, except that the compounds 19-1, 26-1, 19-3, 27-1, 19-2, and 28-1 were used instead of compounds 16-2 and 20-1, and a compound 31-1 synthesized in US-B-8,519,150 was used instead of the compound 20-2.

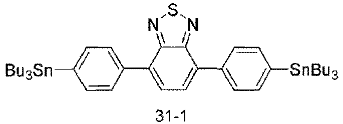

### [Synthesis of polymer B-16]

In Scheme 12, a polymer B-16 was obtained in the same manner as in Scheme 12, except that the compounds 19-1, 26-1, 19-3, 27-1, 19-2, and 28-1 were used instead of the compounds 16-2 and 20-1, and a compound 32-1 synthesized with reference to J. Org. Chem. 2003, Book 68, p. 9813 was used instead of the compound 20-2.

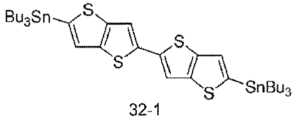

### [Synthesis of polymer B-17]

In Scheme 12, a polymer B-17 was obtained in the same manner as in Scheme 12, except that the compounds 19-1, 26-1, 19-3, 27-1, 19-2, and 28-1 were used instead of the compounds 16-2 and 20-1, and a compound 33-1 synthesized with reference to Macromolecules 2013, Book 46, p. 1337 was used instead of the compound 20-2.

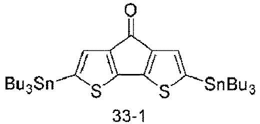

### [Synthesis of polymer B-18]

A polymer B-18 (742 mg) was synthesized from a compound 33-2 synthesized with reference to J. Am. Chem. Soc. 2011, Book 133, p. 11442 by using compounds 19-1/26-1/19-3/27-1/19-2/28-1 (1 g in total) according to Scheme 13 at the reaction temperature of 100°C and the reaction time of three hours.

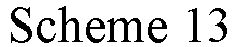

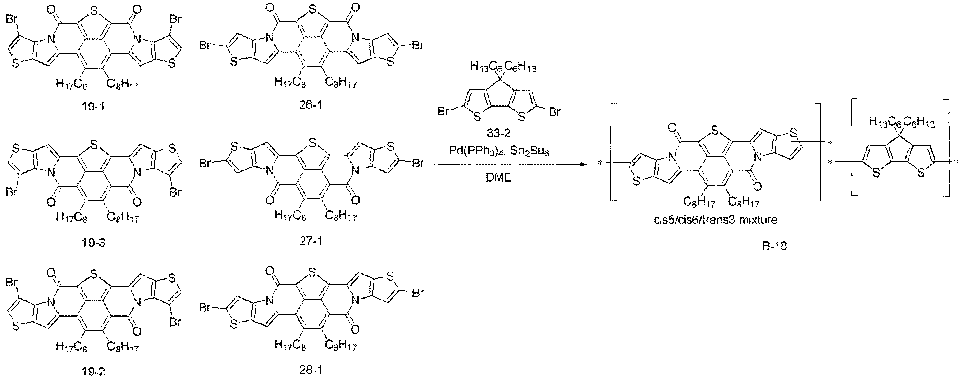

### [Synthesis of polymer B-19]

In Scheme 12, a polymer B-19 was obtained in the same manner as in Scheme 12, except that the compounds 19-1, 26-1, 19-3, 27-1, 19-2, and 28-1 were used instead of the compounds 16-2 and 20-1, and a compound 34-1 synthesized with reference to Tetrahedron Letters 2013, Book 54, p. 2795 was used instead of the compound 20-2.

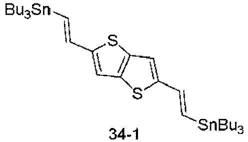

### [Synthesis of polymer B-27]

A polymer B-27 (861 mg) was synthesized from compounds 16-2/20-1/17-2/21-1/17-1/22-1 (1 g in total) according to Scheme 14 at temperature of 100°C and the reaction time of three hours.

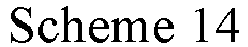

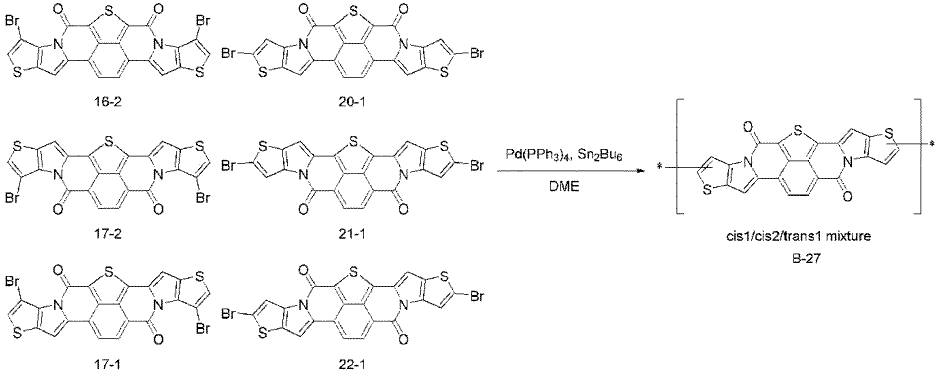

### [Comparative Synthesis Example 2]

Comparative polymers 3 and 4 were synthesized according to the following scheme.

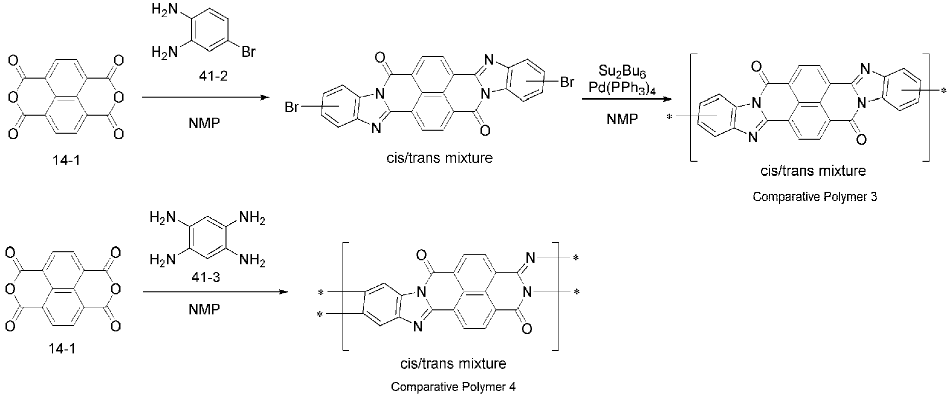

Structures of the obtained compound B-1 to B-27 and the obtained comparative polymer 3 and 4 are provided below. A mixture of a cis isomer and a trans isomer is described using the structure of the trans isomer for convenience of description.

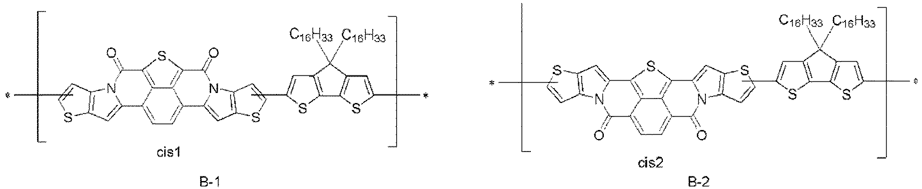

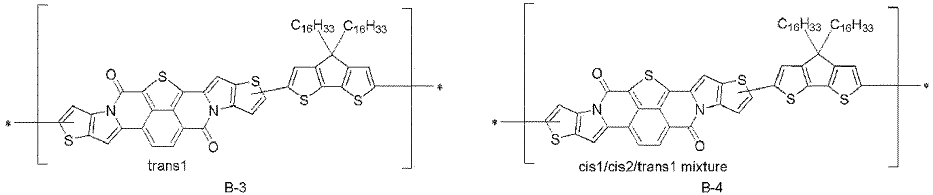

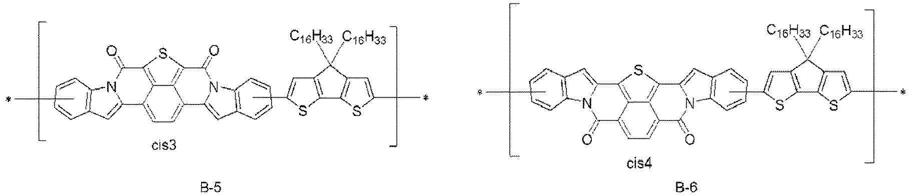

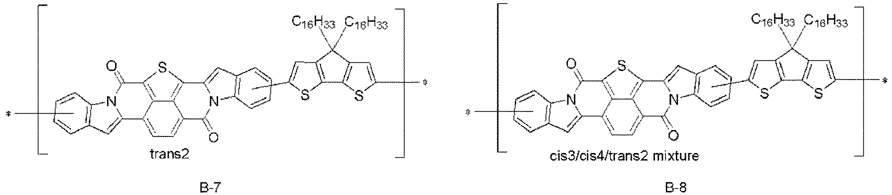

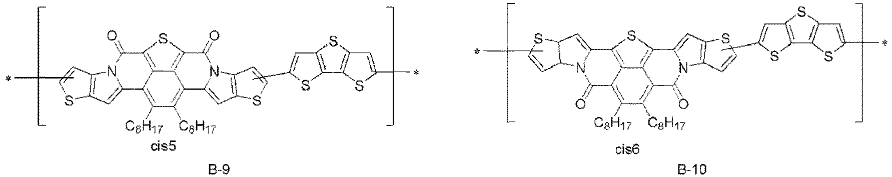

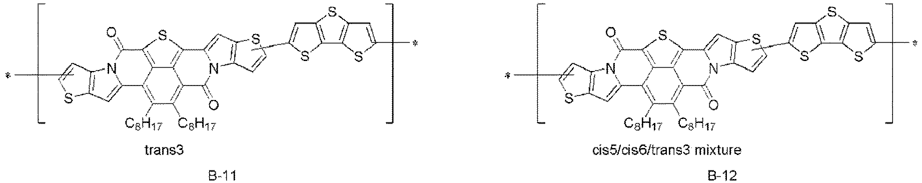

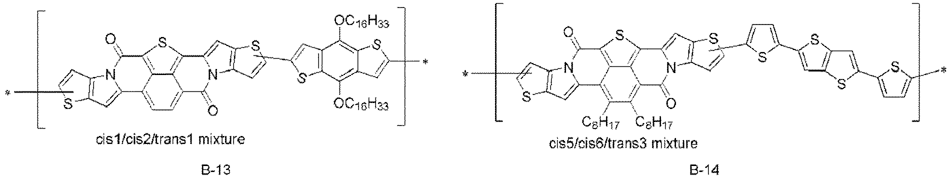

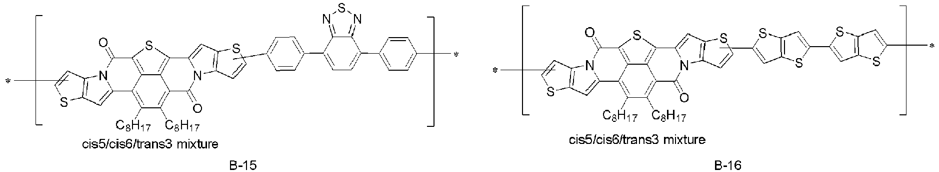

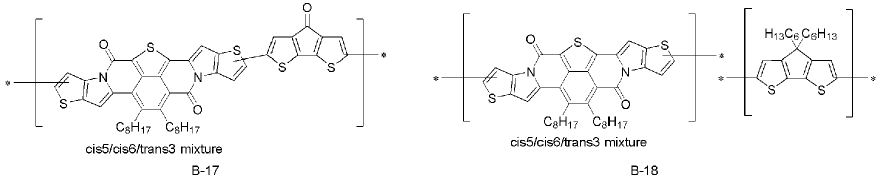

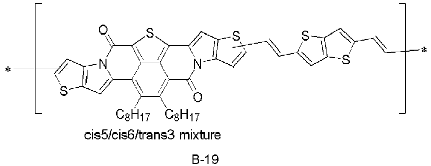

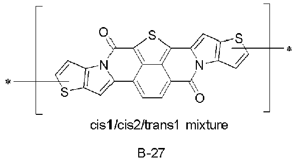

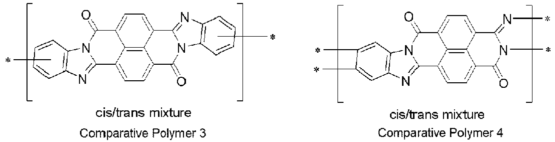

### [Preparation of organic semiconductor composition]

0.1 mass% solution of the polymer B-1 was prepared by mixing the polymer B-1 (a) synthesized above and toluene as a solvent and heated to 40°C, so as to obtain an organic semiconductor polymer composition 1.

The organic semiconductor polymer compositions 2 to 31 and the comparative polymer compositions 1 and 2 were prepared in the same manner, except that each polymer of the polymers B-1 (b) to (e), and B-2 to B-27, and the comparative polymers 3 and 4 was used instead of the polymer B-1(a).

### [Manufacturing of bottom gate-bottom contact type element by coating process]

The organic thin film transistor element 3-1 (hereinafter, referred to as an "element 3-1") was formed by casting (drop cast method) the organic semiconductor polymer composition 1 on a substrate for the field effect transistor (FET) characteristic measurement heated to 40°C in a nitrogen atmosphere.

As the substrate for FET characteristic measurement, a silicon substrate having a bottom gate and bottom contact structure comprising chromium/gold (gate width W = 100 mm, gate length L = 100 µm) disposed in a comb shape as source and drain electrodes and SiO₂ (film thickness of 500 nm) as an insulating film was used.

Organic thin film transistor elements 3-2 to 3-31 (hereinafter, also referred to as "elements 3-2 to 3-31") and comparative organic thin film transistor elements 3-1 and 3-2 (hereinafter, also referred to as "comparative elements 3-1 and 3-2) were manufactured in the same manner as in the manufacturing of the element 3-1 except that each of the organic semiconductor polymer compositions 2 to 31 and the comparative polymer compositions 1 and 2 was used instead of the organic semiconductor polymer composition 1. The obtained elements 3-1 to 3-31 and the obtained comparative elements 3-1 and 3-2 were respectively used as the organic thin film transistor elements in Examples 3-1 to 3-31, and Comparative Examples 3-1 and 3-2.

### [Evaluation of carrier mobility and heat resistance]

With respect to each organic thin film transistor element (the elements 3-1 to 3-31 and the comparative elements 3-1 and 3-2), the carrier mobility and the heat resistance were evaluated in the same manner as in the evaluation of the elements 1-4 to 1-34 and the comparative elements 1-1 and 1-2. The results thereof are presented in the following table.

**[Table 3]**

| | Element No. | Polymer in organic semiconductor layer | | Carrier mobility | Heat resistance |
|---|---|---|---|---|---|
| | | Kind | Mw | | |
| Example 3-1 | Element 3-1 | B-1(a) | 3100 | AA | AA |
| Example 3-2 | Element 3-2 | B-1(b) | 11900 | AA | AA |
| Example 3-3 | Element 3-3 | B-1(c) | 48500 | AA | AA |
| Example 3-4 | Element 3-4 | B-1(d) | 97100 | AA | AA |
| Example 3-5 | Element 3-5 | B-1(e) | 143200 | AA | AA |
| Example 3-6 | Element 3-6 | B-2 | 62500 | AA | AA |
| Example 3-7 | Element 3-7 | B-3 | 56700 | AA | AA |
| Example 3-8 | Element 3-8 | B-4 | 32300 | AA | AA |
| Example 3-9 | Element 3-9 | B-5 | 89100 | A | A |
| Example 3-10 | Element 3-10 | B-6 | 7000 | A | A |
| Example 3-11 | Element 3-11 | B-7 | 13200 | A | A |
| Example 3-12 | Element 3-12 | B-8 | 10500 | A | A |
| Example 3-13 | Element 3-13 | B-9 | 9600 | AA | AA |
| Example 3-14 | Element 3-14 | B-10 | 121400 | AA | AA |
| Example 3-15 | Element 3-15 | B-11 | 74300 | AA | AA |
| Example 3-16 | Element 3-16 | B-12 | 18900 | AA | AA |
| Example 3-17 | Element 3-17 | B-13 | 76400 | AA | AA |
| Example 3-18 | Element 3-18 | B-14 | 143400 | AA | AA |
| Example 3-19 | Element 3-19 | B-15 | 5800 | A | A |
| Example 3-20 | Element 3-20 | B-16 | 33100 | B | B |
| Example 3-21 | Element 3-21 | B-17 | 86700 | C | B |
| Example 3-22 | Element 3-22 | B-18 | 52700 | C | c |
| Example 3-23 | Element 3-23 | B-19 | 36800 | B | A |
| Example 3-31 | Element 3-31 | B-27 | 37500 | D | C |
| Comparative Example 3-1 | Comparative Element 3-1 | Comparative Polymer 3 | 5200 | G | G |
| Comparative Example 3-2 | Comparative Element 3-2 | Comparative Polymer 4 | 7600 | F | F |

As presented in Table 3, even in a case where the organic semiconductor layer was an element including a polymer having a fused polycyclic structure, in a case where the structure of the polymer was out of the range determined in the present invention, the carrier mobility was inferior, and the heat resistance was inferior, as a result (Comparative Examples 3-1 and 3-2).

Meanwhile, it is understood that an element in which a polymer having a structure determined in the present invention was used for the organic semiconductor layer had excellent carrier mobility and also excellent heat resistance (Examples 3-1 to 3-31).

### [Manufacturing of bottom gate-bottom contact type element by flexographic printing]

A coating solution was prepared by dissolving 0.5 mass% of the polymer B-1 (a), 0.5 mass% of poly α-methylstyrene, and 0.05% of BYK 323 (manufactured by BYK-Chemie GmbH) as a surfactant in tetralin, so as to obtain an organic semiconductor polymer composition 4-1. The organic semiconductor polymer compositions 4-2 to 4-31 and the comparative polymer compositions 4-1 and 4-2 were prepared in the same manner, except that each polymer of the polymers B-1 (b) to (e), and B-2 to B-27, and the comparative polymers 3 and 4 was used instead of the polymer B-1 (a).

In the same manner as in the manufacturing of the element 3-1, a bottom gate-bottom contact type substrate for FET characteristic measurement was prepared, and the organic semiconductor polymer composition 4-1 was printed thereon by a flexo printing method, so as to form an organic semiconductor layer. An organic thin film transistor element 4-1 (hereinafter, also referred to as an "element 4-1") was obtained.

Specific method of forming the organic semiconductor layer by a flexographic printing method is as described below.

A flexo aptitude testing machine F1 (manufactured by IDC Testing Systems Co., Ltd.) was used as a printing device, and 1.70% of AFP DSH (manufactured by Asahi Kasei Co., Ltd.) / solid image was used as a flexo resin plate. Printing was performed at the pressure between the printing plate and the substrate of 60 N, and a transportation speed of 0.4 m/sec, and drying was performed at 60°C for two hours so to form an organic semiconductor layer (film thickness: 50 nm).

Organic thin film transistor elements 4-2 to 4-31 (hereinafter, also referred to as "elements 4-2 to 4-31") and comparative organic thin film transistor elements 4-1 and 4-2 (hereinafter, also referred to as "comparative elements 4-1 and 4-2") were respectively manufactured in the same manner as in the manufacturing of the element 4-1 except that each polymer composition of the organic semiconductor polymer compositions 4-2 to 4-31 and the comparative polymer compositions 4-1 and 4-2 was used instead of the organic semiconductor polymer composition 4-1. The obtained elements 4-1 to 4-31, and the obtained comparative elements 4-1 and 4-2 were respectively used as the organic thin film transistor elements in Examples 4-1 to 4-31, and Comparative Examples 4-1 and 4-2.

### [Evaluation of carrier mobility and heat resistance]

With respect to each organic thin film transistor element (the elements 4-1 to 4-31 and the comparative elements 4-1 and 4-2), the carrier mobility and the heat resistance were evaluated in the same manner as in the evaluation of the elements 1-4 to 1-34 and the comparative elements 1-1 and 1-2. The results thereof are presented in the following table.

**[Table 4]**

| | Element No. | Polymer in organic semiconductor layer | | Carrier mobility | Heat resistance |
|---|---|---|---|---|---|
| | | Kind | Mw | | |
| Example 4-1 | Element 4-1 | B-1(a) | 3100 | AA | AA |
| Example 4-2 | Element 4-2 | B-1(b) | 11900 | AA | AA |
| Example 4-3 | Element 4-3 | B-1(c) | 48500 | AA | AA |
| Example 4-4 | Element 4-4 | B-1(d) | 97100 | AA | AA |
| Example 4-5 | Element 4-5 | B-1(e) | 143200 | AA | AA |
| Example 4-6 | Element 4-6 | B-2 | 62500 | AA | AA |
| Example 4-7 | Element 4-7 | B-3 | 56700 | AA | AA |
| Example 4-8 | Element 4-8 | B-4 | 32300 | AA | AA |
| Example 4-9 | Element 4-9 | B-5 | 89100 | A | A |
| Example 4-10 | Element 4-10 | B-6 | 7000 | A | A |
| Example 4-11 | Element 4-11 | B-7 | 13200 | A | A |
| Example 4-12 | Element 4-12 | B-8 | 10500 | A | A |
| Example 4-13 | Element 4-13 | B-9 | 9600 | AA | AA |
| Example 4-14 | Element 4-14 | B-10 | 121400 | AA | AA |
| Example 4-15 | Element 4-15 | B-11 | 74300 | AA | AA |
| Example 4-16 | Element 4-16 | B-12 | 18900 | AA | AA |
| Example 4-17 | Element 4-17 | B-13 | 76400 | AA | AA |
| Example 4-18 | Element 4-18 | B-14 | 143400 | AA | AA |
| Example 4-19 | Element 4-19 | B-15 | 5800 | A | A |
| Example 4-20 | Element 4-20 | B-16 | 33100 | B | B |
| Example 4-21 | Element 4-21 | B-17 | 86700 | C | B |
| Example 4-22 | Element 4-22 | B-18 | 52700 | C | c |
| Example 4-23 | Element 4-23 | B-19 | 36800 | B | A |
| Example 4-31 | Element 4-31 | B-27 | 37500 | D | C |
| Comparative Example 4-1 | Comparative Element 4-1 | Comparative Polymer 3 | 5200 | G | G |
| Comparative Example 4-2 | Comparative Element 4-2 | Comparative Polymer 4 | 7600 | F | F |

As presented in Table 4, even in a case where the organic semiconductor layer was an element including a polymer having a fused polycyclic structure, in a case where the structure of the polymer was out of the range determined in the present invention, the carrier mobility was inferior, and the heat resistance was inferior, as a result (Comparative Examples 4-1 and 4-2).

Meanwhile, it is understood that an element in which a polymer having a structure determined in the present invention was used for the organic semiconductor layer had excellent carrier mobility and also excellent heat resistance (Examples 4-1 to 4-31).

### Explanation of References

10: substrate
20: gate electrode
30: gate insulating film
40: source electrode
42: drain electrode
50: organic semiconductor film
60: sealing layer
100, 200: organic thin film transistor element

## Claims

1. A compound of formula (1) or (2), or a polymer having at least one structural unit of any of formulae (9) and (10): 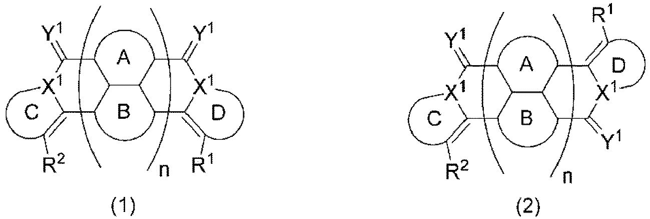 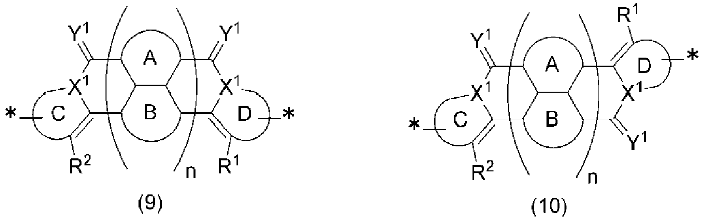 wherein
the fused ring structure formed of rings A and B is a structure of formula (4): 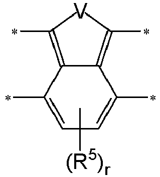 wherein
R⁵ each is halogen or a group ^{∗}-L-T,
V is O, S, Se or NR^{d} wherein R^{d} is H or a substituent,
r is an integer of 0-2, and
^{∗} is a bonding site.
X¹ is N or CR^{a},
rings C, D each are a 5- or 6-membered aromatic or heteroaromatic ring, or a fused ring including the same,
Y¹ is O or S,
R^{a}, R^{b}, R^{c} each are H or a substituent,
n is 1,
^{∗} is a bonding site,
R¹, R² each are halogen or a group ^{∗}-L-T, wherein
^{∗} is a bonding site,
T is H, halogen or cyano, and
L is a single bond, a divalent group of any of the formulae (L-1) to (L-25), or a divalent group formed by bonding two or more divalent groups of any of (L-1) to (L-25): 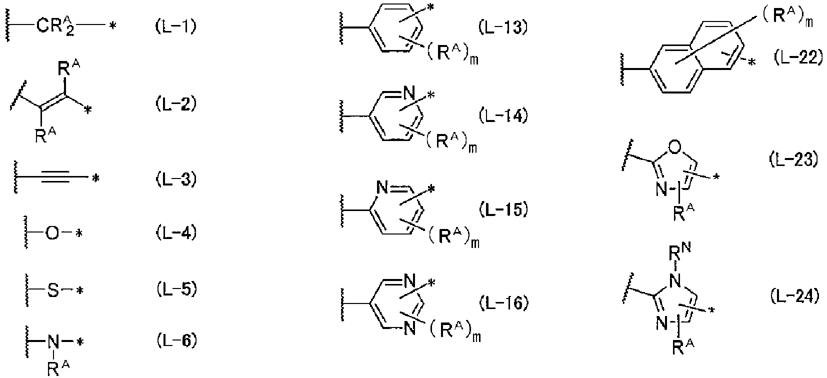 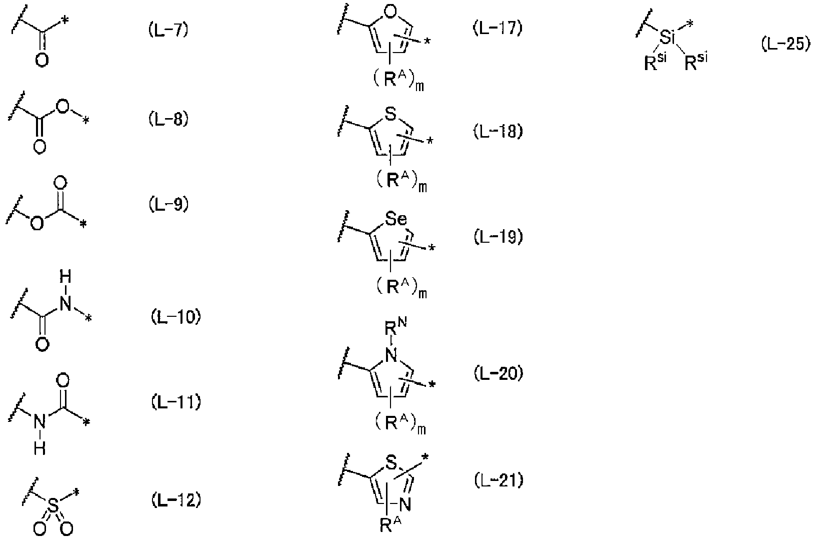
wherein
the wavy line portion is a bonding site to a ring structure of formula (1), (2), (9) or (10), or a bonding site to ^{∗} of a divalent group of any of formulae (L-1) to (L-25),
^{∗} is a bonding site to T, or a bonding site to a wavy line portion of a divalent group of any of Formulae (L-1) to (L-25),
R^{A} is H or a substituent,
R^{N} is H or a substituent,
R^{si} is H, alkyl, alkenyl or alkynyl; and
m is an integer of 1-4 in formula (L-13), is an integer of 1-3 in formulae (L-14) and (L-15), is 1 or 2 in formulae (L-16) to (L-20) and is an integer of 1-6 in formula (L-22).

2. An organic semiconductor element, comprising an organic semiconductor layer containing a compound of formula (1) as defined in claim 1 and/or a compound of formula (2) as defined in claim 1, or containing a polymer having at least one structural unit of any of formulae (9) and (10) as defined in claim 1.

3. The compound or polymer of claim 1 or the organic semiconductor element of claim 2, wherein the rings C and D are fused ring structures of the formula (5) or (6): 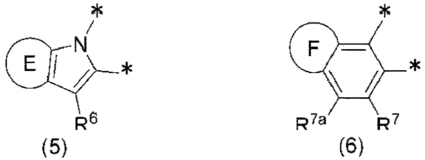 wherein
rings E, F each are a 5- or 6-membered aromatic or heteroaromatic ring, or a fused ring including the same,
R⁶, R⁷, R^{7a} each are H, halogen, or a group ^{∗}-L-T, and
^{∗} is a bonding site,
and in the fused ring structure of formula (5) or (6) in formulae (9) and (10), rings E and F each have one bonding site for being incorporated into a polymer chain.

4. The organic semiconductor element of claim 3, wherein the fused ring structure of formula (5) is a structure of formula (7) or (8): 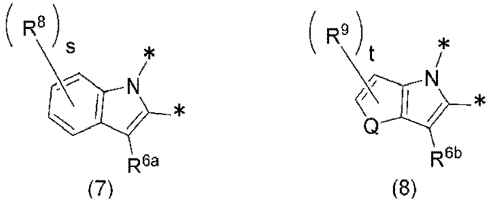 wherein
R^{6a}, R^{6b} each are H, halogen or a group ^{∗}-L-T,
R⁸, R⁹ each are halogen or a group ^{∗}-L-T,
s is an integer of 0-4,
t is an integer of 0-2,
Q is a chalcogen atom, and
^{∗} is a bonding site,
and in the fused ring structure of formula (7) or (8) in formulae (9) and (10), one of ring-constituting atoms of a ring which may have R⁸ or R⁹ has a bonding site to be incorporated into a polymer chain.

5. The organic semiconductor element of claim 4, wherein the rings C and D are fused ring structures of formula (8), and in the fused ring structure of formula (8) in formulae (9) and (10), one of the ring-constituting atoms of the ring which may have R⁹ has one bonding site for being incorporated into a polymer chain.

6. The compound or polymer of claim 1 or the organic semiconductor element of any of claims 2-5, wherein L is a divalent group selected from the formulae (L-1) to (L-4), (L-13), (L-17) and (L-18), or a group obtained by bonding two or more of these groups.

7. The compound or polymer of claim 1 or the semiconductor element of any of claims 2-6, wherein the polymer has a structure of formula (G):
^{∗}-Ar¹-(Vr)ₚ₃-Ar²-^{∗} (G)
wherein
Ar¹, Ar² each are a single bond, vinylene, ethynylene, arylene or heteroarylene, or a divalent group formed by linking two or more of these groups; and preferably each are a single bond or a divalent group of formula (Ar-1) or (Ar-2): 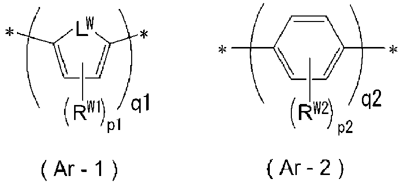
wherein
R^{W1} is alkyl, and p1 is an integer of 0-2,
L^{W} is a chalcogen atom,
R^{W2} is alkyl, and p2 is an integer of 0-4,
q1, q2 each are an integer of 1-4, and
^{∗} is a bonding site,
Vr is a divalent conjugated C₂₋₄₀-group, and
p3 is an integer of 1-6, and preferably is 1.

8. The organic semiconductor element of claim 7, wherein the polymers alternately have a structural unit of any of formulae (9) and (10) and a structure of formula (G).

9. The organic semiconductor element of claim 7 or 8, wherein Vr in formula (G) is a structure selected from formulae (V_{A}-1) to (V_{A}-11) and (V_{D}-1) to (V_{D}-16), and preferably is a divalent group of any of formulae (V_{D}-1) to (V_{D}-16): 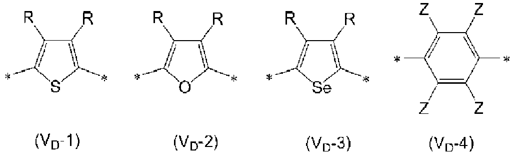 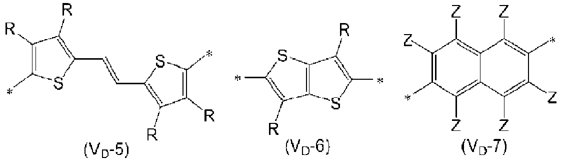 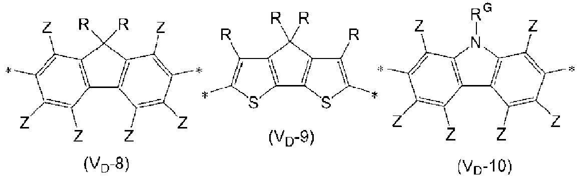 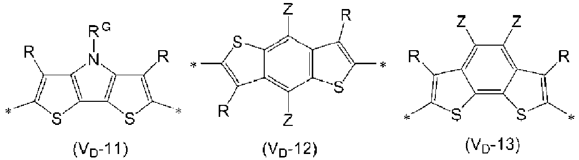 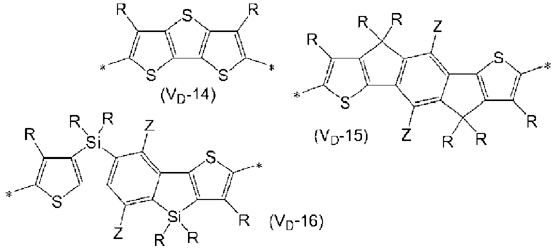 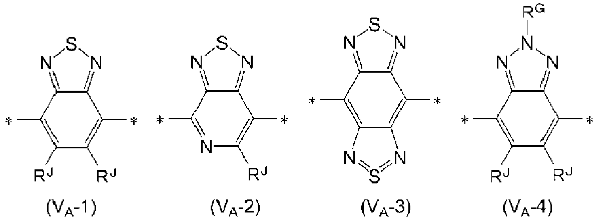 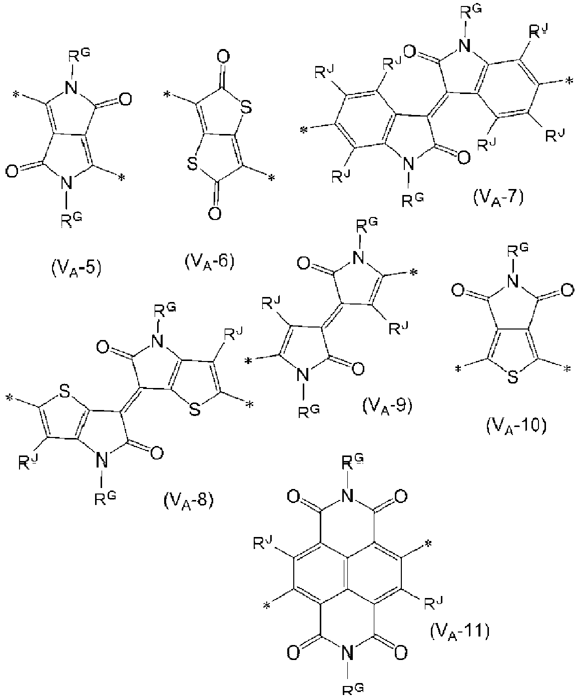 wherein R and Z each are H, halogen or alkyl; R^{G} is alkyl; R^{J} is H, alkyl, cyano or halogen; and ^{∗} is a bonding site.

10. The organic semiconductor element of any of claims 2-9, which is an organic thin film transistor element.

11. An organic semiconductor composition comprising a compound of formula (1) as defined in claim 1 and/or a compound of formula (2) as defined in claim 1, or a polymer having at least one structural unit of any of formulae (9) and (10) as defined in claim 1; a solvent; and optionally a binder.

12. An organic semiconductor film comprising a compound of formula (1) as defined in claim 1 and/or a compound of formula (2) as defined in claim 1 or a polymer having at least one structural unit of any of formulae (9) and (10) as defined in claim 1.

13. A method for manufacturing an organic semiconductor film, comprising coating a substrate with the organic semiconductor composition of claim 11 to form a coating film; and drying the coating film.

## Patentansprüche

1. Verbindung der Formel (1) oder (2), oder Polymer mit zumindest einer Struktureinheit gemäß irgendeiner der Formeln (9) und (10): 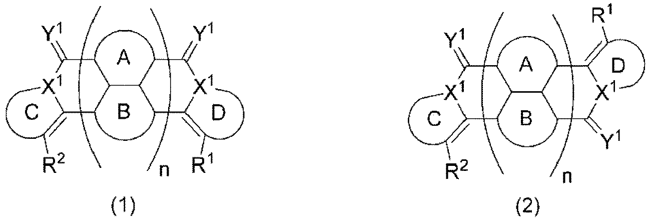 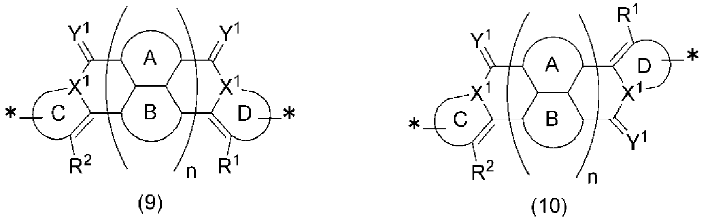 worin
die von den Ringen A und B gebildete kondensierte Ringstruktur eine Struktur der Formel (4) ist: 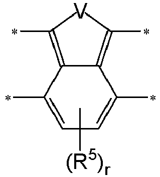 worin
R⁵ jeweils Halogen oder eine Gruppe *-L-T ist,
V O, S, Se oder NR^{d} ist, worin R^{d} H oder ein Substituent ist,
r eine ganze Zahl von 0-2 ist und
* eine Bindungsstelle ist;
X¹ N oder CR^{a} ist,
die Ringe C und D jeweils ein 5- oder 6-gliedriger aromatischer oder heteroaromatischer Ring oder ein kondensierter Ring, der diesen umfasst, sind,
Y¹ O oder S ist
R^{a}, R^{b}, R^{c} jeweils H oder ein Substituent sind,
n 1 ist,
* eine Bindungsstelle ist,
R¹, R² jeweils Halogen oder eine Gruppe *-L-T sind, worin
* eine Bindungsstelle ist,
T H, Halogen oder Cyano ist und
L eine Einfachbindung, eine divalente Gruppe gemäß irgendeiner der Formeln (L-1) bis (L-25) oder eine divalente Gruppe, gebildet durch Binden von zwei oder mehr divalenten Gruppen von (L-1) bis (L-25), ist: 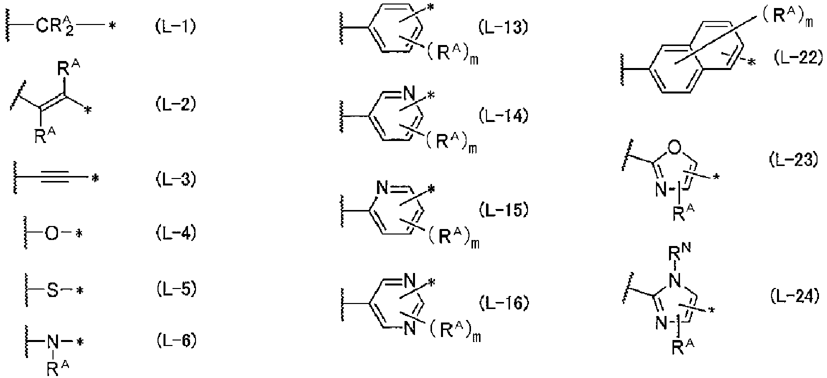 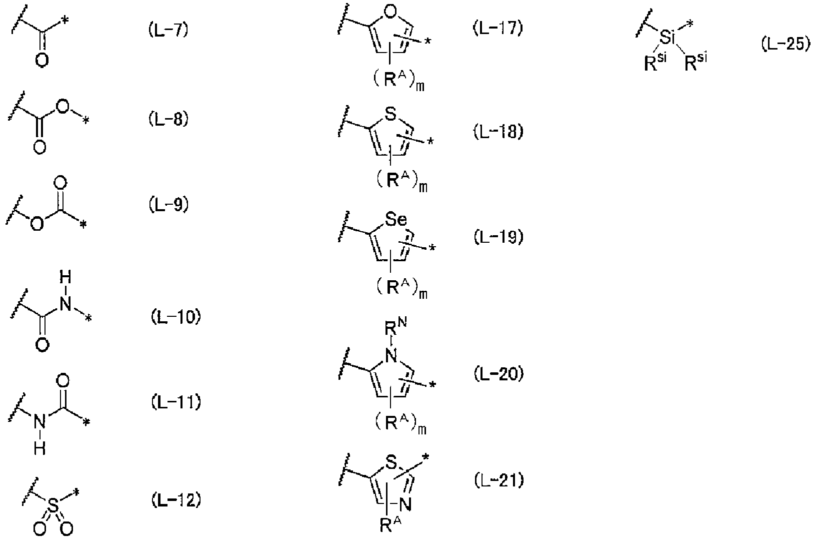
worin
der gewellte Linienteil eine Bindungsstelle an eine Ringstruktur mit der Formel (1), (2), (9) oder (10) oder eine Bindungsstelle an * einer divalenten Gruppe gemäß irgendeiner der Formeln (L-1) bis (L-25) ist,
* eine Bindungsstelle an T oder eine Bindungsstelle an einen gewellten Linienteil einer divalenten Gruppe gemäß irgendeiner Formeln (L-1) bis (L-25) ist,
R^{A} H oder ein Substituent ist,
R^{N} H oder ein Substituent ist,
R^{si} H, Alkyl, Alkenyl oder Alkinyl ist; und
m eine ganze Zahl von 1-4 in der Formel (L-13), eine ganze Zahl von 1-3 in der Formel (L-14) und (L-15), 1 oder 2 in den Formel (L-16) bis (L-20) und eine ganze Zahl von 1-6 in der Formel (L-22) ist.

2. Organisches Halbleiterelement, umfassend eine organische Halbleiterschicht, die eine wie in Anspruch 1 definierte Verbindung der Formel (1) und/oder eine wie in Anspruch 1 definierte Verbindung der Formel (2) enthält, oder die ein Polymer mit zumindest einer Struktureinheit gemäß irgendeiner der in Anspruch 1 definierten Formeln (9) und (10) enthält.

3. Verbindung oder Polymer gemäß Anspruch 1 oder organisches Halbleiterelement gemäß Anspruch 2, worin die Ringe C und D kondensierte Ringstrukturen der Formel (5) oder (6) sind: 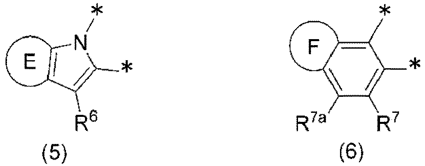 worin
die Ringe E, F jeweils ein 5- oder 6-gliedriger aromatischer oder heteroaromatischer Ring oder ein kondensierter Ring, der diesen umfasst, sind,
R⁶, R⁷, R^{7a} jeweils H, Halogen oder eine Gruppe *-L-T sind und
* eine Bindungsstelle ist,
und in der kondensierten Ringstruktur der Formel (5) oder (6) in den Formeln (9) und (10) die Ringe E und F jeweils eine Bindungsstelle aufweisen, die sie in eine Polymerkette einbindet.

4. Organisches Halbleiterelement gemäß Anspruch 3, worin die kondensierte Ringstruktur der Formel (5) eine Struktur der Formel (7) oder (8) ist: 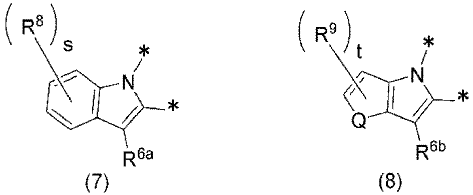 worin
R^{6a}, R^{6b} jeweils H, Halogen oder eine Gruppe *-L-T sind,
R⁸, R⁹ jeweils Halogen oder eine Gruppe *-L-T sind,
s eine ganze Zahl von 0-4 ist,
t eine ganze Zahl von 0-2 ist
Q ein Chalcogenatom ist und
* eine Bindungsstelle ist,
und worin in der kondensierten Ringstruktur der Formel (7) oder (8) in den Formeln (9) und (10) eines der Ringaufbauenden Atome eines Rings, der R⁸ oder R⁹ aufweisen kann, eine Bindungsstelle zur Einbindung in eine Polymerkette aufweist.

5. Organisches Halbleiterelement gemäß Anspruch 4, worin die Ringe C und D kondensierte Ringstrukturen der Formel (8) sind und in den kondensierten Ringstrukturen der Formel (8) in den Formeln (9) und (10) eines der Ringaufbauenden Atome des Rings, der R⁹ aufweisen kann, eine Bindungsstelle zur Einbindung in eine Polymerkette aufweist.

6. Verbindung oder Polymer gemäß Anspruch 1 oder organisches Halbleiterelement gemäß irgendeinem der Ansprüche 2-5, worin L eine divalente Gruppe ist, ausgewählt aus den Formeln (L-1) bis (L-4), (L-13), (L-17) und (L-18), oder eine Gruppe ist, die durch Binden von zwei oder mehr dieser Gruppen erhalten wird.

7. Verbindung oder Polymer gemäß Anspruch 1 oder organisches Halbleiterelement gemäß irgendeinem der Ansprüche 2-6, worin das Polymer eine Struktur der Formel (G) aufweist:
*-Ar¹-(Vr)ₚ₃-Ar²-* (G)
worin
Ar¹, Ar² jeweils eine Einfachbindung, Vinylen, Ethinylen, Arylen oder Heteroarylen oder eine divalente Gruppe, gebildet durch Verknüpfen von zwei oder mehr dieser Gruppen, sind, und bevorzugt jeweils eine Einfachbindung oder eine divalente Gruppe der Formel (Ar-1) oder (Ar-2) sind: 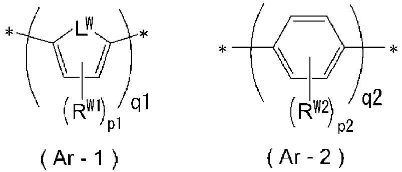
worin
R^{W1} Alkyl ist und p1 eine ganze Zahl von 0-2 ist,
L^{W} ein Chalcogenatom ist,
R^{W2} Alkyl ist und p2 eine ganze Zahl von 0-4 ist,
q1, q2 jeweils eine ganze Zahl von 1-4 sind und
* eine Bindungsstelle ist,
Vr eine divalente konjugierte C₂₋₄₀-Gruppe ist und
p3 eine ganze Zahl von 1-6, und bevorzugt 1, ist.

8. Organisches Halbleiterelement gemäß Anspruch 7, worin die Polymere alternierend ein Struktureinheit gemäß irgendeiner der Formeln (9) und (10) und eine Struktur der Formel (G) aufweisen.

9. Organisches Halbleiterelement gemäß Anspruch 7 oder 8, worin Vr in der Formel (G) eine Struktur ist, die ausgewählt ist aus den Formeln (V_{A}-1) bis (V_{A}-11) und (V_{D}-1) bis (V_{D}-16), und bevorzugt eine divalente Gruppe gemäß irgendeiner der Formeln (V_{D}-1) bis (V_{D}-16) ist: 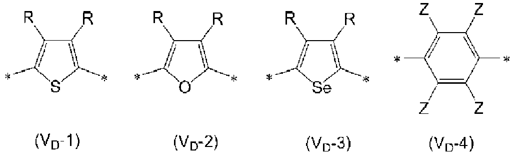 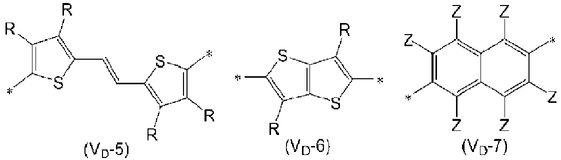 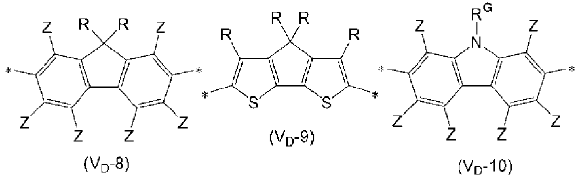 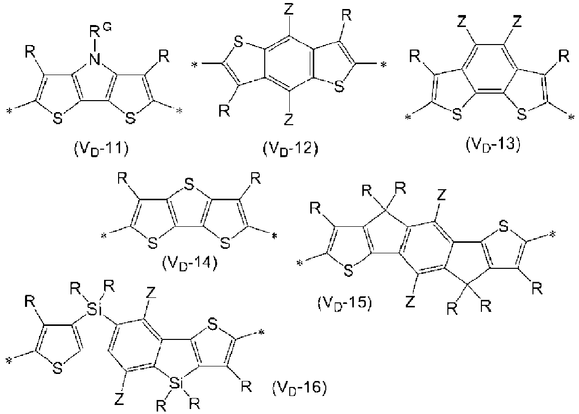 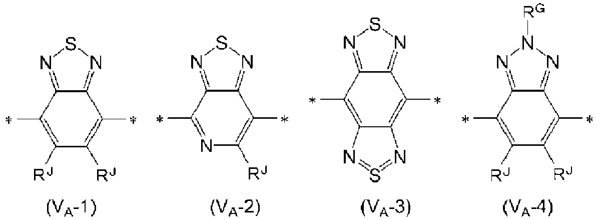 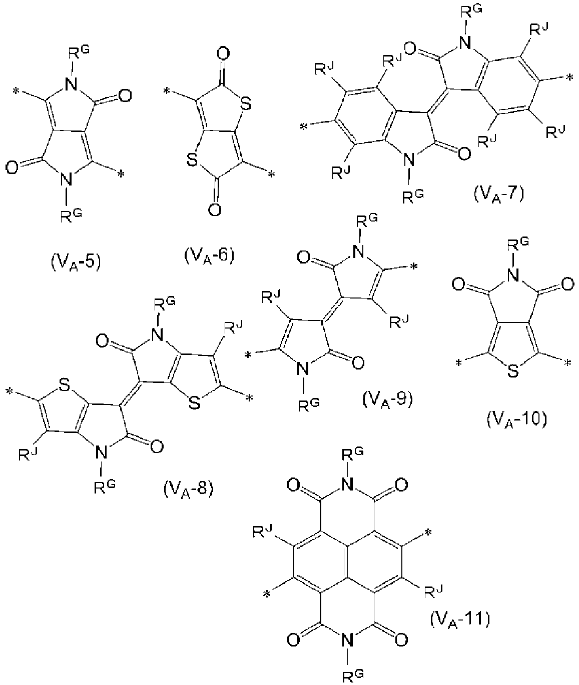 worin R und Z jeweils H, Halogen oder Alkyl sind, R^{G} Alkyl ist, R^{J} H, Alkyl, Cyano oder Halogen ist; und * eine Bindungsstelle ist.

10. Organisches Halbleiterelement gemäß irgendeinem der Ansprüche 2 bis 9, welches ein organisches Dünnfilm-Transistorelement ist.

11. Organische Halbleiterzusammensetzung, umfassend eine wie in Anspruch 1 definierte Verbindung der Formel (1) und/oder eine wie in Anspruch 1 definierte Verbindung der Formel (2), oder ein Polymer mit mindestens einer Struktureinheit gemäß irgendeiner der wie in Anspruch 1 definierten Formeln (9) und (10); ein Lösungsmittel; und optional ein Bindemittel.

12. Organischer Halbleiterfilm bzw. -folie, umfassend eine wie in Anspruch 1 definierte Verbindung der Formel (1) und/oder eine wie in Anspruch 1 definierte Verbindung der Formel (2), oder ein Polymer mit zumindest einer Struktureinheit gemäß irgendeiner der wie in Anspruch 1 definierten Formeln (9) und (10).

13. Verfahren zur Herstellung eines organischen Halbleiterfilms oder bzw. -folie, umfassend das Beschichten eines Substrats mit der organischen Halbleiterzusammensetzung gemäß Anspruch 11, um einen Beschichtungsfilm zu bilden; und Trocknen des Beschichtungsfilms.

## Revendications

1. Composé de formule (1) ou (2), ou polymère comportant au moins une unité structurelle de l'une quelconque des formules (9) et (10) : 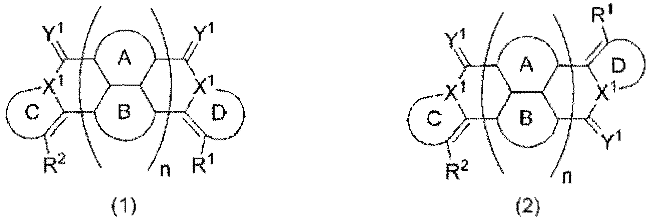 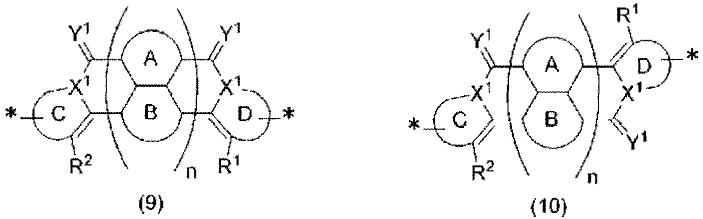 dans lequel
la structure à cycles accolés constituée des cycles A et B est une structure de formule (4) : 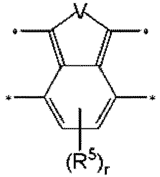 dans lequel
R⁵ chacun étant un halogène ou un groupe ^{∗}-L-T,
V est O, S, Se ou NR^{d} dans lequel R^{d} est H ou un substituant,
R est un entier de 0-2, et
^{∗} est un site de liaison.
X¹ est N ou CR^{a},
cycles C, D chacun étant un cycle aromatique ou hétéro-aromatique à 5 ou 6 membres, ou un cycle accolé incluant celui-ci,
Y¹ est O ou S,
R^{a}, R^{b}, R^{c} chacun étant H ou un substituant,
n est 1,
^{∗} est un site de liaison,
R¹, R² chacun étant un halogène ou un groupe ^{∗}-L-T, dans lequel
^{∗} est un site de liaison,
T est H, halogène ou cyano, et
L est une liaison unique, un groupe divalent de l'une quelconque
des formules (L-1) à (L-25), ou un groupe divalent constitué par la liaison de deux groupes divalents ou plus de l'une quelconque des formules (L-1) à (L-25) : 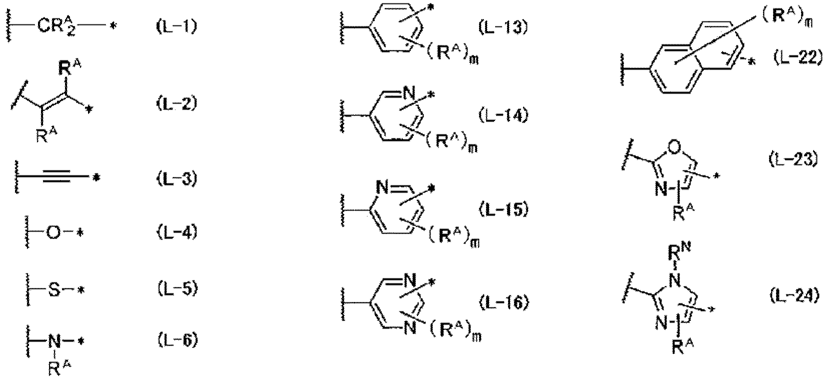 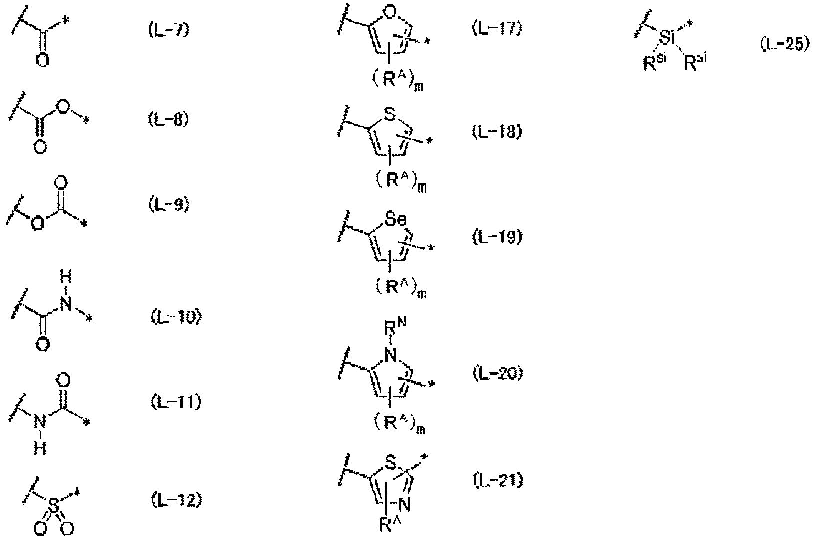 dans lequel
la partie de ligne ondulée est un site de liaison à une structure de cycle de la formule (1), (2), (9) ou (10), ou un site de liaison à ^{∗} d'un groupe divalent de l'une quelconque des formules (L-1) à (L-25),
^{∗} est un site de liaison à T, ou un site de liaison à une partie de ligne ondulée d'un groupe divalent de l'une quelconque des formules (L-1) à (L-25),
R^{a} est H ou un substituant,
R^{N} est H ou un substituant,
R^{si} est H, alkyle, alkényle ou alkynyle ; et
m est un entier de 1-4 dans la formule (L-13), est un entier de 1-3 dans les formules (L-14) et (L-15), est 1 ou 2 dans les formules (L-16) à (L-20) et est un entier de 1-6 dans la formule (L-22).

2. Élément semi-conducteur organique, comprenant une couche de semi-conducteur organique contenant un composé de formule (1) selon la revendication 1 et/ou un composé de formule (2) selon la revendication 1, ou contenant un polymère comportant au moins une unité structurelle de l'une quelconque des formules (9) et (10) selon la revendication 1.

3. Composé ou polymère selon la revendication 1 ou élément semi-conducteur selon la revendication 2, dans lequel les cycles C et D sont des structures à cycles accolés de la formule (5) ou (6) : 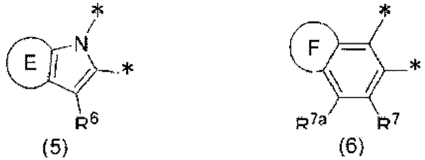 dans lequel
cycles E, F chacun étant un cycle aromatique ou hétéro-aromatique à 5 ou 6 membres, ou un cycle accolé incluant celui-ci,
R⁶, R⁷, R^{7a} chacun étant H, halogène, ou un groupe ^{∗}-L-T, et
^{∗} est un site de liaison,
et dans la structure à cycles accolés de formule (5) ou (6) dans les formules (9) et (10), les cycles E et F comportent chacun un site de liaison pour être incorporé dans une chaîne polymère.

4. Élément semi-conducteur organique selon la revendication 3, dans lequel la structure à cycles accolés de formule (5) est une structure de formule (7) ou (8) : 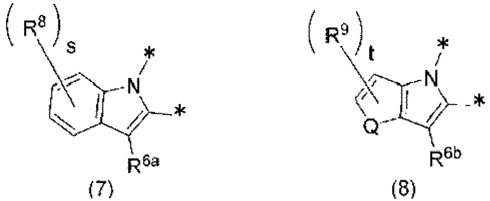 dans lequel
R^{6a}, R^{6b} chacun étant H, halogène ou un groupe ^{∗}-L-T,
R⁸, R⁹ chacun étant halogène ou un groupe ^{∗}-L-T,
s est un entier de 0-4,
t est un entier de 0-2,
Q est un atome de chalcogène, et
^{∗} est un site de liaison,
et, dans la structure à cycles accolés de formule (7) ou (8) dans les formules (9) et (10), l'un d'atomes constitutifs de cycle d'un cycle pouvant comporter R⁸ ou R⁹ comporte un site de liaison à incorporer dans une chaîne polymère.

5. Élément semi-conducteur organique selon la revendication 4, dans lequel les cycles C et D sont des structures à cycles accolés de formule (8), et, dans la structure à cycles accolés de formule (8) dans les formules (9) et (10), l'un d'atomes constitutifs de cycle d'un cycle pouvant comporter R⁹ comporte un site de liaison à incorporer dans une chaîne polymère.

6. Composé ou polymère selon la revendication 1 ou élément semi-conducteur organique selon l'une quelconque des revendications 2 à 5, dans lequel L est un groupe divalent sélectionné parmi les formules (L-1) à (L-4), (L-13), (L-17) et (L-18), ou un groupe obtenu par la liaison de deux de ces groupes ou plus.

7. Composé ou polymère selon la revendication 1 ou élément semi-conducteur organique selon l'une quelconque des revendications 2 à 6, dans lequel le polymère comporte une structure de formule (G) :
^{∗}-Ar¹-(Vr)_{P3}-Ar²-^{∗} (G)
dans lequel
Ar¹, Ar² chacun étant une liaison unique, vinylène, éthynylène, arylène ou hétéroarylène, ou un groupe divalent constitué par la liaison de deux de ces groupes ou plus; et préférentiellement chacun étant une liaison unique ou un groupe divalent de formule (Ar-1) ou (Ar-2) : 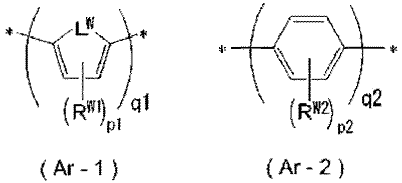 dans lequel
R^{W1} est alkyle, et pl est un entier de 0-2,
L^{W} est un atome de chalcogène,
R^{W2} est alkyle, et p2 est un entier de 0-4,
q1, q2 chacun étant un entier de 1-4, et
^{∗} est un site de liaison,
Vr est un groupe divalent conjugué C₂-₄₀, et
p3 est un entier de 1-6, et préférentiellement est 1.

8. Élément semi-conducteur organique selon la revendication 7, dans lequel les polymères comportent en alternance une unité structurelle de l'une quelconque des formules (9) et (10) et une structure de formule (G).

9. Élément semi-conducteur organique selon la revendication 7 ou 8, dans lequel Vr dans formule (G) est une structure sélectionnée parmi les formules (V_{A}-1) à (V_{A}-11) et (V_{D}-1) à (V_{D}-16), et préférentiellement est un groupe divalent de l'une quelconque des formules (V_{D}-1) à (V_{D}-16) : 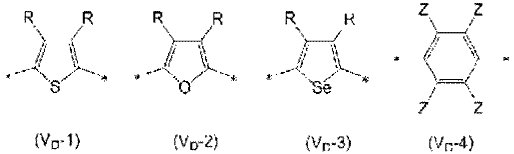 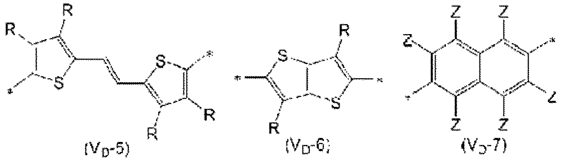 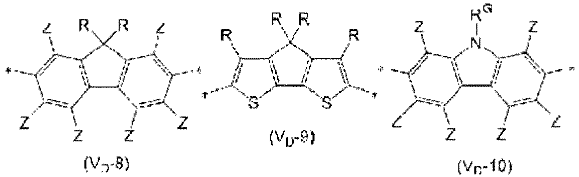 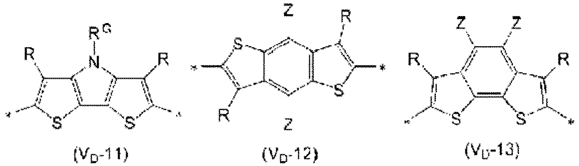 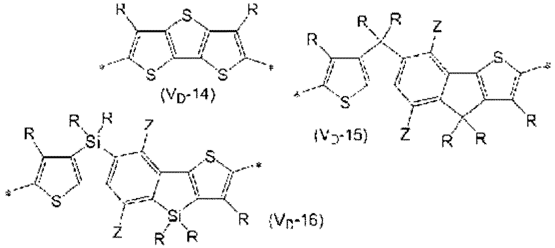 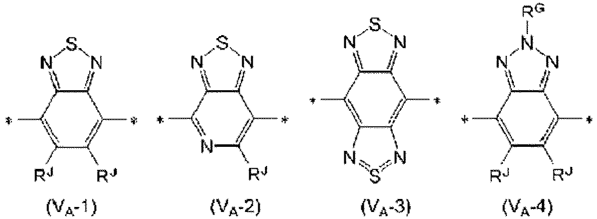 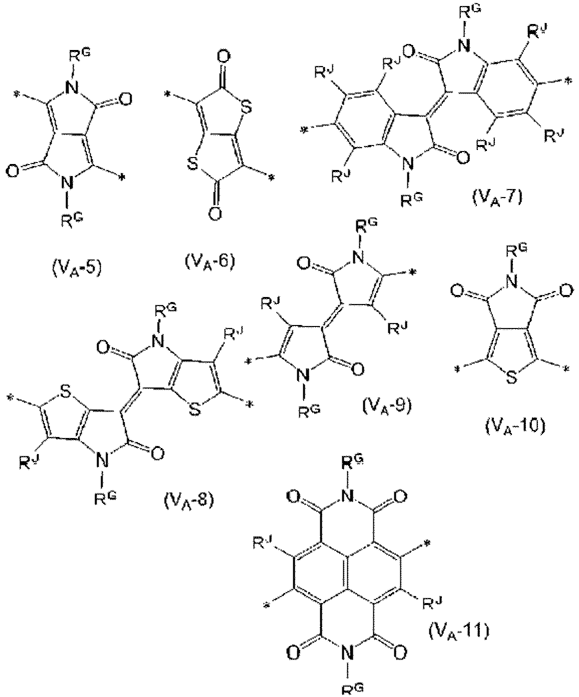 dans lequel R et Z sont chacun H, halogène ou alkyle ; R^{G} est alkyle ; R^{J} est H, alkyle, cyano ou halogène ; et ^{∗} est un site de liaison.

10. Élément semi-conducteur organique selon l'une quelconque des revendications 2 à 9, qui est un élément transistor à film mince organique.

11. Composition de semi-conducteur organique comprenant un composé de formule (1) selon la revendication 1 et/ou un composé de formule (2) selon la revendication 1, ou un polymère comportant au moins une unité structurelle de l'une quelconque des formules (9) et (10) selon la revendication 1 ; un solvant; et facultativement un liant.

12. Film de semi-conducteur organique comprenant un composé de formule (1) selon la revendication 1 et/ou un composé de formule (2) selon la revendication 1, ou un polymère comportant au moins une unité structurelle de l'une quelconque des formules (9) et (10) selon la revendication 1.

13. Procédé de fabrication d'un film de semi-conducteur organique, comprenant le revêtement d'un substrat avec la composition de semi-conducteur organique selon la revendication 11 pour former un film de revêtement; et le séchage du film de revêtement.
